# EUROPEAN PATENT SPECIFICATION

(11) **EP 3 245 601 B1**
(45) Date of publication and mention of the grant of the patent: **16.06.2021**
(21) Application number: 16738030.2
(22) Date of filing: 16.01.2016
(51) Int. Cl.: G16H 50/20, G16H 10/60, G06Q 50/22

(54) **HEALTHCARE DATA INTERCHANGE SYSTEM AND METHOD**
SYSTEM UND VERFAHREN ZUM AUSTAUSCH VON GESUNDHEITSDATEN
SYSTÈME ET PROCÉDÉ D'ÉCHANGE DE DONNÉES DE SOINS DE SANTÉ

(30) Priority: 16.01.2015 US 201562104532 P
(43) Date of publication of application: 22.11.2017
(73) Proprietor: PricewaterhouseCoopers LLP, New York, NY 10017 (US)
(72) Inventor: MYNHIER, Mark, Anaheim, CA 92807 (US); PATHAK, Dhiraj, Great Neck, NY 11021 (US)
(74) Representative: Potter Clarkson
(86) International application number: PCT/US2016/013754
(87) International publication number: WO 2016/115551

(56) References cited:
- US-A1- 2007 050 446
- US-A1- 2010 082 370
- US-A1- 2012 271 612
- US-A1- 2013 024 125
- US-A1- 2014 201 095
- US-A1- 2014 244 309
- US-A1- 2014 297 395
- US-B2- 8 744 815

## Description

### BACKGROUND

Many countries and states within the United States are facing an impending healthcare crisis due to declining population health, increasing prevalence of chronic diseases, and the extraordinarily high cost of caring for patients in an acute care system. Current disease management programs rely primarily on manual, siloed interventions, which are labor intensive and un-scalable.

US 2014/0201095 discloses a healthcare assurance system in which a data integrity engine, with one or more processors, may check health information to ensure quality of data underlying particular preventative, curative, palliative, and/or other care recommendations for a particular patient wherein the data integrity engine may assess each piece of information underlying a recommendation and may assign a weight to the information according to a score that is assigned according to how important such information is to prevent redundant, harmful or unnecessary care service recommendations. This prior art does not suggest any mechanism to ensure effective matching with patient records for data integration into a common information model.

### SUMMARY

The present disclosure relates to a health data system that is part of a scalable technology core that can be integrated into local healthcare infrastructure to create a care management framework for delivering patient-centric and value-based care in a community, setting the stage for scalability to targeted communities.

In accordance with a first aspect disclosed herein, there is set forth a health data system for delivering patient-centric and value-based care, comprising:
a health data server;
one or more health data sources in communication with the health data server over a secured network, wherein said health data sources each have a set of polling permissions and wherein said data sources comprise body monitor devices;
one or more agent modules of the health data server that poll health data from the data sources at a designated frequency based on a set of identifiers and the set of polling permissions;
a first switch module for providing the polled health data into a common information model, the common information model being defined by at least one patient record, each patient record having a plurality of attributes, and wherein the first switch module is configured to match the polled health data from the two or more data sources to the at least one patient record using a total match score, the total match score based on a sum of two or more weighted scores applied to the attributes, wherein the attributes of the polled health data are assigned predetermined weights determined based on the likelihood of change throughout a patient's life and, upon matching, the polled health data is tagged with a unique identifier for the patient in the health data system; and
one or more interface modules for gaining access to the common information model based on a set of access permissions.

In some embodiments of the disclosed system, the common information model includes a distributed database and wherein the one or more attributes optionally define at least one of clinical health data, laboratory data, remote monitoring data, biometrics, wearables, social media data, self-reported data, mobile application data, and device instrumentation.

In some embodiments of the disclosed system, the first switch module further augments the common information model with a new attribute when the polled health data does not map into the one or more attributes.

In some embodiments of the disclosed system, the first switch module at least one of filters the polled health data prior to providing the polled health data into the common information model based on storage permissions, the storage permissions optionally being provided by at least one of the health data server and the one or more health data sources, matches patient records against each other, and controls network connections over the secured network.

In some embodiments of the disclosed system, the designated frequency is set by at least one of the health data server and the one or more health data sources.

In some embodiments of the disclosed system, the system includes a second switch module for providing the polled health data into the common information model, wherein said first switch module communicates with said second switch module for receiving the polled health data.

In accordance with another aspect disclosed herein, there is set forth a method for delivering patient-centric and value-based care, the method comprising:

polling one or more health data sources for health data via one or more agent modules of a health data server, wherein each of the one or more health data sources have a set of polling permissions and wherein the two or more health data sources are in communication with the health data server over a secured network, and wherein said polling is performed at a designated frequency based on a set of identifiers and the set of polling permissions; and wherein said data sources comprise body monitor devices;
populating a common information model with the polled health data via a first switch module, the common information model being defined by at least one patient record, each patient record having one or more attributes and wherein the method includes matching the polled health data from the two or more data sources to the at least one patient record using a total match score, the total match score based on a sum of two or more weighted scores applied to the attributes, wherein the attributes of the polled health data are assigned predetermined weights determined based on the likelihood of change throughout a patient's life and, upon matching, the polled health data is tagged with a unique identifier for the patient in the health data system; and
providing access to the common information model via one or more interface modules based on a set of access permissions.

In some embodiments of the disclosed method, populating the common information model comprises populating a distributed database optionally with the one or more attributes selected from at least one of clinical health data, laboratory data, remote monitoring data, biometrics, wearables, social media data, self-reported data, mobile application data, and device instrumentation.

In some embodiments of the disclosed method, the method further comprises augmenting the common information model with a new attribute when the polled health data does not map into the one or more attributes via the first switch module.

In some embodiments of the disclosed method, the method further comprises filtering the polled health data based on a set of storage permissions prior to said populating.

In some embodiments of the disclosed method, the set of storage permissions are provided by at least one of the health data server and the one or more health data sources.

In some embodiments of the disclosed method, polling occurs at a designated frequency set by at least one of the health data server and the one or more health data sources.

In some embodiments of the disclosed method, the method further comprises polling a second switch module via the first switch module for the polled health data.

In some embodiments of the disclosed method, polling is limited by the set of polling permissions.

### BRIEF DESCRIPTION OF THE DRAWINGS

Fig. 1 is an exemplary network diagram illustrating an embodiment of a health data system.
Fig. 2 is an exemplary network diagram illustrating an alternative embodiment of the health data system of Fig 1.
Fig. 3 is an exemplary flow chart illustrating an embodiment of a method of obtaining and processing healthcare data by the health data system of Figs. 1 and 2.
Fig. 4 is an exemplary flow chart illustrating an embodiment of a method of processing and providing healthcare data by the health data system of Figs. 1 and 2.
Fig. 5 is an exemplary method of defining a consumer health problem, creating an ecosystem to resolve it, monitoring the care delivery network to identify where health benefits are realized, and isolating the associated cost savings in order to redistribute them to various stakeholders.
Fig. 6 is an exemplary network diagram illustrating an embodiment of health care ecosystem.

It should be noted that the figures are not drawn to scale and that elements of similar structures or functions are generally represented by like reference numerals for illustrative purposes throughout the figures. It also should be noted that the figures are only intended to facilitate the description of the preferred embodiments. The figures do not illustrate every aspect of the described embodiments and do not limit the scope of the present disclosure.

### DETAILED DESCRIPTION OF THE PREFERRED EMBODIMENTS

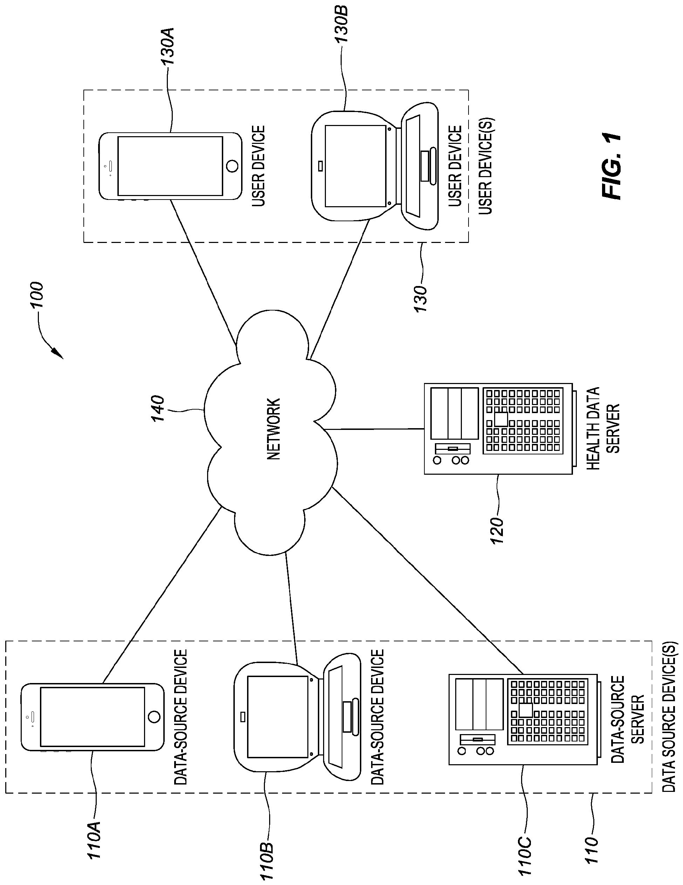

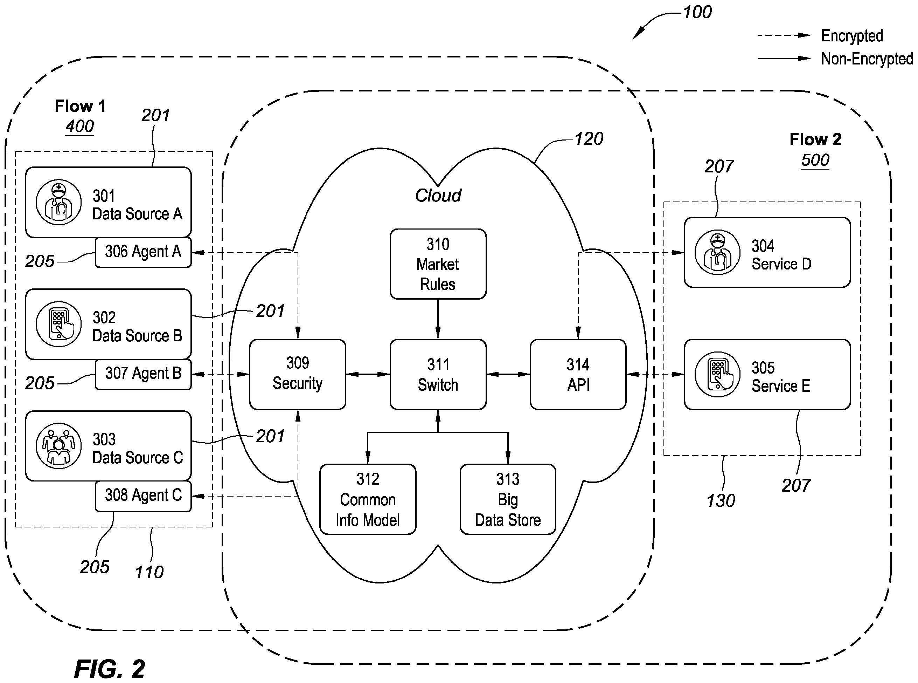

Emerging technology solutions are poised to transform health care delivery. The health data system 100 as illustrated in Figs. 1 and 2 can be used along with other systems to implement a suite of technology-enabled data-driven solutions designed to augment and accelerate effective disease management and care. For example, the health data system 100 can be part of a scalable technology core that can be integrated into local healthcare infrastructure to create a care management framework for delivering patient-centric and value-based care in a community, setting the stage for scaling to a broader set of communities.

In various embodiments it can be beneficial to configure a system that is consumer-centric with mobility and access, self-service enabled and designed with data fluidity and continuity. It can also be beneficial to have a personalized system that is configured to treat a person, not a diagnosis (e.g., by using a contextualized health profile and by leveraging longitudinal physiological plus behavioral, social and environmental data). It can be further beneficial to have an outcome-based system wherein value is driven by health outcomes; where quality is defined by safe and evidence-based care; where efficiency is achieved through optimized allocation of capacity, capability, availability and cost; and where effectiveness is personalized based on personal preference and ability, impacted by social and environmental factors. A desirable system can also include proactive health management that extends beyond reactive episodic care, includes population segmentation and stratification, includes a chronic disease care coordination plan, includes a long term health management plan, and includes a consumer education plan.

A health data system 100 that provides for obtaining, storing, curating, analyzing and/or providing access to health data can prove desirable and provide a basis for a wide range of applications as described in detail herein. This result can be achieved, according to one embodiment disclosed herein, by a health data system 100 as illustrated in Fig. 1.

Turning to Fig. 1, the health data system 100 is shown as comprising a plurality of data-source devices 110, a health data server 120, and a plurality of user devices 130. In some embodiments, the user devices 130 represent user services available from the health data system 100. The data-source devices 110 and user devices 130 are connected to the health data server 120 by a secure network 140 that can consist of any combination of wireless and wireline links. The data-source devices 110 are shown as comprising a smart phone data-source 110A, a laptop data-source 110B and a server data-source 110C, but in further embodiments, any suitable device can comprise a data-source 110, including a desktop computer, a tablet computer, a gaming device, a smart-television, a headset computer, a smartwatch, a body monitor device, or the like. Additionally, various embodiments can include any suitable number of any such data-source devices 110.

Similarly, although the user devices 130 are shown as being invoked from a smart phone user device 130A and a laptop user device 130B, in further embodiments, a user device 130 can comprise any suitable device including a server, a desktop computer, a tablet computer, a gaming device, a smart-television, a headset computer, a smartwatch, a body monitor device, or the like. Additionally, various embodiments can include any suitable plurality of any such user devices 130.

The server 120 can include one or more server systems, which can include any suitable plurality of devices and/or a cloud-based system. Additionally, the server 120 can comprise a plurality of modules, databases, or the like. For example, Fig. 2 illustrates one embodiment of the health data system 100 that comprises one or more agent modules 205, a security module 309, a market rules module and/or database 310, a switch module 311, a common info model module and/or database 312, a big data store 313 and an API 314, which are part of a cloud-based server system 120. The cloud-based server system 120 shown in Fig. 2 is implemented as a private cloud for illustration purposes only.

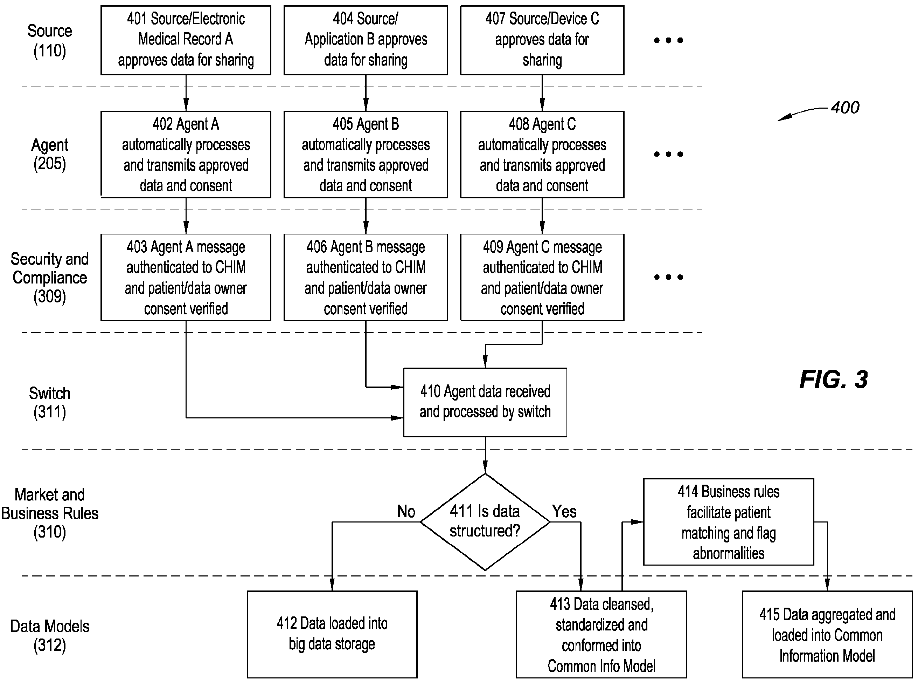

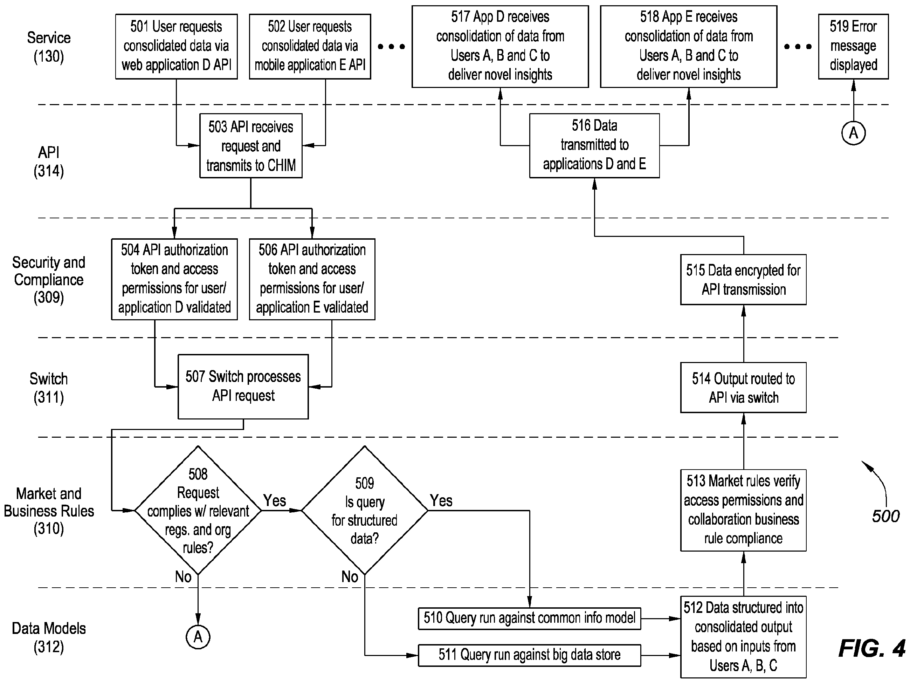

In accordance with various embodiments, the server 120 is configured to receive, process and store data obtained from data source devices 110 (see, *e.g.,* Fig. 3). The server 120 can also be configured to process and/or retrieve stored data and provide it to the one or more user devices 130 in response to various queries or data requests that the user devices 130 may provide (see, *e.g.,* Fig. 4).

Referring to Figs. 1 and/or 2, one or more data source device 110 can be associated with one or more data source 201. For example, data sources 201 can include a variety of potential stakeholders and their associated data sources that might approve data for sharing via the health data system 100 in a targeted health ecosystem. For example, data sources 201 can include healthcare providers (e.g., data can be electronic medical records, lab data), health insurers / payers (e.g., data can be claims records), pharmaceutical and medical device companies *(e.g.,* data can be clinical trial records, adverse event data), research *(e.g.,* data can be a genomic profile), government/community health programs (e.g., data can be population health statistics) partnership databases), and/or individual patients (e.g., data can be biometrics, activity/behavior). Each data source 201 can be independently owned with its own set of unique data access control rules. Accordingly, the health data system 100 advantageously provides access to disparately held data (e.g., across the data sources 201) for use by independently developed health data services (e.g., via the user devices 130).

Each stakeholder data source 201 can choose the specific fields and elements, or subsets of data, which they approve to share, and the system 100 can manage the approvals of identified data (*e.g.,* through consent/data use agreement) and/or identifiable data (*e.g.,* through consent/ Business Associate Agreements) as described in more detail herein and as illustrated in Fig. 3. Although examples of data associated with a given data source are described above, data sources can provide or be the source of any suitable type of data without limitation.

Agents A-C 306, 307, and 308 of Fig. 2 represent an example architecture of the distributed agent modules 205 that are configured to obtain, receive and/or access data on a manual and/or automated basis from the data sources 201. The agent modules 205 can be associated with the one or more data sources 201 and/or the data source devices 110, and a given data source 201 or data source device 110 can be associated with one or more agent modules 205. These agent modules 205 ensure that any needed metadata / supporting elements (e.g., consents, access rights, source information) are transmitted alongside data that is obtained, received and/or accessed from the data sources 201.

In some embodiments, manual interactions can be conducted via a web-enabled portal within the data source 201 (*e.g.,* via one or more data source device 110), in which an owner of the data source 201 is responsible for deciding what data is transmitted to the server 120 from the data source 201 by agent modules 205. Automated interactions can be via script programs that are configured (*e.g*., with business rules, or the like) and then scheduled to run on a particular frequency, and/or in real time based on desired monitoring criteria to specify what data is transmitted to the server 120 from the data source 201 by the agent modules 205. The data in the data source 201 that can be transmitted to the server 120 by the agent modules 205 is designated by an identifier. An identifier is a string of alpha-numeric characters that uniquely identify a patient record in the data source 201.

Business rules can be configured in the agent modules 205 and can include naming conventions, data lineage tracking, permissible and non-permissible fields based on the ability to share identifiable data, sharing restrictions, and other stakeholder organization-specific rules for the agent to follow when managing data. In some embodiments, as illustrated in Fig. 2, agent modules 205 can encrypt data that will be transmitted to the server 120 prior to leaving environment and/or firewall of data source 201.

In various embodiments, data that is not in accordance with the sharing business rules defined by the data source 201 will remain in the environment of the data source 201 and will not be brought into the health data system 100 by the agent modules 205. In some embodiments, this can include identifiable data that does not contain a flag indicating that a patient's consent for sharing was obtained and a notice of privacy practices was given, such that the health data system 100 must infer that the data source 201 does not have permission from an individual to share the individual's data outside of the environment of the data source 201 (*e.g.,* blocks 403, 406 and 409 of Fig. 3).

In one embodiment, the implementation of a selected agent module 205 includes the following steps:
1. The agent module 205 is designated with the IP address of the data device 110 that holds the data provided by a data source 201.
2. The agent module 205 is designated with identifiers for patient records available from the data source 201. The identifiers are provided by the data source 201 to the health data server 120.
3. The agent module 205 is designated with a frequency for obtaining data on patient records from the data source 201 (using the identifiers set in step 2). The frequency may be set by the health data server 120 and/or the health data sources 201.
4. The secure network 140 establishes a dedicated connection between the health data system 100 and the data source 201.
5. The agent module 205 collects data from the data source 201 on the identifiers (set in step 2), from the location (set in step 1) per designated frequency (set in step 3) using the dedicated network connection (set in step 4).
6. Data collected by the agent module 205 is stored in a message queue in the health data server 120. This message queue is processed by the switch 311. The agent module 205 represents the data collected from the data source as a list of (<attribute>, <value>) pairs.
7. The agent module 205 continues to run until the health data server 120 is notified by the data source 201 to no longer collect data from the data source 201.
8. At any time the data source 201 can modify the list of identifiers on whom data can be collected by the agent module 205 for transmission to the server 120.
9. At any time the data source 201 can send a message to the health data server 120 to terminate data available from the data sources 201 in the health data server 120 on one or more (or all) identifiers on whom the agent module 205 may have collected data from the data source 201 (in step 5).
10. At any time the data source can obtain from the health data server 120 a report on what data was collected by agent module 205 from the data source 201.

As shown in Fig. 2, the server 120 can comprise a security module 309, which can comprise one or more security components that are operable to ensure communications to and from the server 120 are authorized/authenticated and/or encrypted in accordance with data source stakeholder needs, regulatory requirements, and industry best practices *(see, e.g.,* blocks 403, 406 and 409 of Fig. 3). For example, the security module 309 and components thereof can be configured to ensure that any agent 205 or direct user interactions with the health data server 120 are in compliance with applicable business rules or the like. In some embodiments, the security module 309 can comprise physical and/or virtual security components, which can include the following components, or the like.

### Security Incident and Event Management:

- Log Collection, Correlation and Notification
- External Internet Attack & Threat Monitoring
- Internal Attack & Threat Monitoring

### Identity & Access Management:

- API Gateway Firewall
- Identity Access Management (IAM)
- Advanced Multi Factor Authentication
- Fine Grained Entitlement Access Control
- Privileged Access Management

### Database Security:

- Masking of database information from administrators
- Database Auditing and Accounting of Access
- Database Encryption

### Network Security:

- Unified Threat Management (UTM) Firewalls
- Data Leak Prevention
- Configuration Management & Monitoring
- Vulnerability Management Scanning
- 3^{rd} Party Digital Certificates
- Attack & Penetration Tests
- Load Balancer

### End Point Security:

- Hypervisor VM Firewall Security
- Encryption Vault Key Protection
- Patch Management Protection
- Malware Protection

The health data server 120 can include a market rules module 310 that can control a portion of or all data flow that occurs within the system 100 (*see, e.g.,* blocks 411 and 414 of Fig. 3). A rule in the market rules module 310 specifies access to some data in server 120 from data source 201 by the user device 130.

In one embodiment, market rules module 310 is characterized by the following steps:
1. To configure the market rules module 310 with data access rules, called market rules, for each data source 201 connected to the health data system 100, set up a configuration file containing rules provided to health data server 120 by data source 201
2. A market rule has the following specification:
   a. (<source id> <record id> <service id> <user id>): this rule states that user device 130 identified by <service id> can have access to a record, identified by <record id>, provided by data source 201, identified by <source id>, and available for access by a user of the user device 130, identified by <user id>.
3. To validate a request for data by user device 130 the market rules module 310 checks if the specific record requested by the user device 130 from data source 201 is allowed by the market rules specified by data source 201. A market rule can be specified to inform the switch 311 to not persist data from 201 collected by 205 in the common information model 312. This market rule can have the following specification: (<source id> <record id> on-demand): this rule states that a record identified by <record id> from the data source 201 identified by <source id> should not be stored in the common information model 312. For records from the data source 201 identified by this rule, the switch module 311 stores a query in the common information model 312 for this record and executes this query at the time a request is made for this record by the API 314.

In various embodiments, business and/or market rules can facilitate the application of algorithms and logic such as data consistency checking and cleansing, reference data standardization, and master patient indexing, in order to facilitate reuse and avoid duplication or mis-representation of data. In various embodiments, this can include operations such as identification and adjusting of null values and inconsistencies in units of measure, and the usage of demographic values to align multiple stakeholders' records for the same individual under a single global identifier within the system 100. Accordingly, in some embodiments, business and/or market rules can enable the use of data across a plurality of services to derive novel insights while also protecting information privacy.

The health data server 120 can also include one or more switch modules 311. In one embodiment the implementation of a switch module 311 includes:
1. A selected switch module 311 is set up with an input message queue. The input message queue receives messages from the agent module 205. The format of each message can include a list of (<attribute>, <value>) pairs.
2. The selected switch module 311 is set up with a data quality queue to log messages identifying records obtained from the data source 201 that have been found to have data quality issues by the data quality engine (DQE). DQE includes:
   a. Data quality rules for each data source 201 connecting into system 100
   b. Data quality rule can include the following format: <attribute> <type> <possible values>
   c. Application of data quality rules corresponding to the data source 201 to messages placed into the input message queue of the switch module 311 by the agent module 205 to flag a data quality issue if any attribute of the message in the input message queue does not have a value contained in the set of possible values for that attribute in the data quality rules for that data source 201.
3. The health data server 120 sends to the data source 201 data quality messages from the data quality queue for resolution by the data source 201
4. If no data quality issue is detected with the message in the input message queue then the switch module 311 loads up the data in the message into the corresponding table in the common information model 312. For example, if the message is about the medication administered to a given patient at a hospital then the contents of this message are loaded into the medication table in common information model 312 tagged with the identifier for that patient along-with the identifier of the data source 201 associated with the hospital from where the agent module 205 collected the data for that patient. At the time of loading the data into the common information model 312, one or more coding dictionaries can be used to map terms in the message into standard terms. For example, blood pressure can be mapped to hypertension.
5. The switch module 311 periodically executes the match engine. Match engine detects when data from two different data sources 201 belongs to the same patient.
6. The switch module 311 is set up with an output message queue. Output message queue receives messages from the API module 314.
7. The switch module 311 invokes the market rules module 310 for each message in its output message queue. If the market rules module 310 validates the message for access to requested data from 312 then the switch module 311 maps the message in the output message queue into a query for common information model 312. The query format is (<source id>, <record id>) where <source id> identifies a data source 201 and <record id> identifies a record from source 201. This query is then executed against common information model 312 by switch component 311. The data obtained from common information model 312 by switch 311 is returned to the user device 130 by the API 314.
8. The switch module 311 applies a natural language processing (NLP) engine to unstructured attributes in data from the data sources 201 collected by the agent modules 205 (unstructured attributes are identified in 312 as attributes that allow arbitrary length character strings as values). The resulting data includes (<attribute>, <value>) lists and these lists are used to add data to the corresponding tables in the common information model 312. In this way the switch component 311 reduces unstructured data from 201 into structured data in the common information model 312. By doing so, the switch component 311 integrates structured data from the data sources 201 with unstructured data from the data sources 201.
9. The switch module 311 tags each data stored in the common information model 312 with any data used in the generation of the data being stored in the common information model 312. This way complete data traceability is maintained in the common information model 312. For example, if there two sets of medication records on a patient in 312 and a user service 130 generates a reconciled medication list then prior to storing the reconciled medication list in the common information model 312, the switch module 311 tags the reconciled medication list with the identifiers for the two medication list that have been reconciled.
10. The switch module 311 implements a data exclusion service to de-identify data from the data sources 201. This service is provided with a list attributes that need to be de-identified. Upon execution of the data exclusion service on specified data the values of the identified attributes are masked to make it impossible to obtain the original values of these attributes by replacing each byte of storage allowed for holding a value for an attribute with the null byte '0'.
   Simultaneously, the data is tagged with the names of attributes that are masked. In this way the input data is rendered de-identified by the data exclusion service. In an alternative embodiment of data exclusion service the value of a de-identified attribute can be replaced with a unique tag and the correspondence between the original value of the attribute and the replacement tag can be added to a tokenization table in the common information model 312.

The switch component 311 can implement various services to manage the lifecycle of data from onboarding to termination, including:
- Registration service to onboard & register the data source 201 and the data service 130. The registration of the data source 201 with system 100 requires the data source 201 to provide an interface to system 100. This interface consists of an IP address and a set of instructions on how to obtain data from that IP address. The data source 201 provides a set of data access rules to health data server 120 that govern the use of the data provided by the data source 201 by the user devices 130 participating in system 100. In health data server 120, these rules are called market rules configured in the market rules module 310. Registration by data service with the system 100 requires system 100 to provide to the data service 130 an IP address and a set of instructions on how to obtain data from that IP address. Before responding to a request for data by a registered data service 130 system 100 validates the request against market rules provided by data sources 130 who have contributed requested data into system 100. This way access to data in system 100 is always controlled by data access rules established by the data sources 201 contributing data into system 100.
- Data audit service to log data entering and leaving the system 100.
- Data quality engine (DQE) to monitor the quality of data entering the system 100 which in turn drives better analytics, which can include the following dimensions: Attribute-level data quality (for example age should be in a certain range or last name should not be blank), Context or aggregate data quality (for example patient data on average should be approximately 50% male 50 % female plus or minus certain margin), Operational data quality (for example, reject if patient demographics data is present but medication data is not present thereby rendering the entire patient record not useful)
- Data termination service to remove from the system 100 any data from a data source upon request by the data source owner makes it easy for the source owner to remove their data with control from the platform.
- In some embodiments, where the rules cannot sufficiently resolve inconsistencies in the data, those elements/fields are flagged for manual intervention from a data steward user who is authorized to see identifiable data from all stakeholders and reconcile the differences. This can include examples in which patient matching / indexing algorithms have identified two records, from two stakeholders, which records appear to belong to the same patient but are missing.
- Patient match service to identify and match data on the same patient ingested from multiple data sources 201. For example, using patient record elements that are less likely to change throughout a patient's life (*e.g.,* name, date of birth, gender, SSN-as opposed to a phone number or address for example), the health data server 120 can match a patient across multiple records of different types from different sources. Accordingly, in some embodiments, the health data server 120 can be configured to assign a weight to each field to create a total match score, with the weight implying the importance of that field in the matching process (e.g., social security number (SSN) and date of birth (DOB) can get a 35% weight while gender, last name, and first name can get 10% weight each), which weighting may be operable to improve accuracy of the match. Upon matching data from two or more data sources 201, the switch module 311 tags the data with a unique id for that patient in the health data system 100.

The resulting cleansed, structured, standardized data can be stored in one or more databases defined by the common information model module 312, (see, *e.g.,* block 413 and 415 of Fig. 3). For example, the common information model module 312 can standardize information in accordance with terminologically robust standards such as Systematized Nomenclature of Medicine--Clinical Terms (SNOMED) standards (*e.g*., for procedure codes, medication method), International Organization for Standardization (ISO) standards (*e.g*., for Country Codes), Fast Healthcare Interoperability Resources (FHIR) standards (for certain fields), or the like. For example, medication route and dosage method can be standardized to the SNOMED values, country code can be standardized to 3 digit ISO-3166 country codes, gender and race can be standardized using FHIR standard values, or the like.

In some embodiments, when standardizing and integrating data, the system 100 can also facilitate patient matching. For example, using patient record elements that are less likely to change throughout a patient's life (*e.g.,* name, date of birth, gender, SSN-as opposed to a phone number or address for example), the system 100 can match a patient across multiple records of different types from different sources. Accordingly, in some embodiments, the system 100 can be configured to assign a weight to each field to create a total match score, with the weight implying the importance of that field in the matching process (*e.g*., social security number (SSN) and date of birth (DOB) can get a 35% weight while gender, last name, and first name can get 10% weight each), which weighting may be operable to improve accuracy of the match. Additionally, the business/market rules can also identify unstructured data and prepare it for storage in clusters separate from the rest of the structured data (*e.g.,* in big data storage 313) without losing lineage information (*see, e.g.,* block 412 of Fig. 3).

Although various embodiments discussed herein relate to processing data for storage on the health data server 120, in further embodiments, data processing described herein can apply to data in-motion. In other words, in some embodiments, data may not be stored on the server 120 and can be passed between data sources 201 and data consumers 207 using the health data server 120 as an intermediary, but without the data being stored in the health data server 120. In such embodiments, data can be processed as described herein.

Referring to Figs. 1, 2, and 4, the system 100 can comprise a plurality of data consumers 207 that are configured to consume data from the server 120 for the purpose of research, clinical care, commercial purposes, or the like, using the one or more user devices 130. For example, a given data consumer 207 can comprise one or more user devices 130 that are configured to request and/or receive various types of data from the server 120 as described in more detail herein and as shown in Fig. 4, through the user devices 130.

In various embodiments, data consumers 207 can include healthcare providers (*e.g*., physician from a hospital sharing of electronic medical records with a primary care physician for continuity of care, or the like), health insurers/payers/risk-bearing entities (*e.g*., using outcomes data to adjust formulary status for pharmaceuticals), pharmaceutical and/or medical device companies (*e.g*., using outcomes data to support post-marketing surveillance evaluations / Stage IV clinical trials), government/community health programs (*e.g*., using provider data to support at-home care, especially as it relates to environmental factors), and/or individual patients (*e.g*., medication compliance reminders), who have agreed protect data and use it in accordance with a data use agreement and/or business associate agreement.

Although Fig. 2 shows data sources 201 being separate from data consumers 207, in some embodiments a data source 201 and data consumer 207 can be the same. Additionally, a data source device 110 can also include a user device 130, and vice versa.

In various embodiments, data consumers 207 can request data either manually (*e.g*., via mobile application request initiated by a provider, patient, advocate, or the like) or via automated requests (*e.g.*, automated pull from the health data server 120 common information model database 312 into a local research database (not shown) on a daily, weekly, monthly basis, or the like).

In various embodiments, the application programming interface (API) 314 can be configured to handle requests for data from the data consumers 207 according to a published catalog of service calls (e.g., requests for patient demographics, or queries for various subsets of data). Such interactions can require authorization and authentication, as facilitated by the security components 309. In some embodiments, requests from the API 314 can be managed by the switch 311 according to the market/business rules module 310. Applicable rules can grant or block access for certain requests, based on the level of access associated with each request.

For example, a request for identifiable data by the user device 130 can be blocked if the requesting user (or user's organization/role type) has not been granted rights by the original data source 201 or patient who shared that data (*e.g.,* as configured in the agent 205 that handled the data). In another example, rules can also specify that only an organization's own users can see identifiable data, while all others may only receive access to identified versions or subsets of the organization's data (see, *e.g.,* block 508 in Figure 4). Depending on the nature of the access restriction, the user may receive an error message (see, *e.g.,* block 519 in Figure 4).

In some embodiments, the server 120 can determine whether a request is granted by assessing each request based on three checks:
- Regulatory Compliance: does the request follow Health Insurance Portability and Accountability Act (HIPAA) standards and/or Health Information Trust Alliance (HITRUST) regulations. In other words the health data server 120 checks that proper regulatory documentation is in place (*e.g.,* a HIPAA business associate agreement (BAA)).
- Security: the health data server 120 ensures authentication and authorization.
- Market Rules: the health data server 120 implements organization-specific controls that govern data access rules.

For example, compliant requests/data output, serviced by the API module 314, can include a user requesting access to data for which he or she is an authorized user; the data source 201 requesting that their data on the health data server 120 platform is terminated; a patient requesting that their data not be shared; a patient requesting that certain specific parties only can see their data; a Fast Healthcare Interoperability Resources (FHIR) data output of patient demographics data for a Medical Record Number (MRN) for which the requestor has been granted; a request for Tableau (Tableau, Inc., Seattle WA) visualization of age demographics of multiple patients being viewed by a user who is authorized to access these multiple patients; a request for analytics comparing a patient to other "patients like me" across organizations through a third party clinical decision support solutions; and the like.

Examples of non-compliant requests, serviced by the API module 314, (for which no data output is provided) can include: an individual or organization requesting access to data for which they are not supposed to have access; a request to bypass security policies and procedures (requesting to share user id for example); a request to disable or change a market rule from an unauthorized individual; request to be able to direct Structured Query Language (SQL) query against health data server 120 data stores; a request to use information in a way that falls outside the scope of the data use agreement; a business entity requesting access to protected health information on a patient without a HIPAA business associate agreement (BAA); and the like.

However, if permissions are granted, then the applicable query can be run against the common information model 312 *(see, e.g.,* blocks 509, 510, 511 in Figure 4).

As an example, the output of the common informational query might be a data set in relational format, while the output of the big data query might be a frequency and sentiment analysis for a bolus of unstructured content and specific keywords therein.

The result of the query or queries can be consolidated into a unified output (*see e.g.,* block 512 in Figure 4), to the extent it is not already aggregated, that can be reflective of the combined data of various contributing data source 201 stakeholders, as filtered or defined by the user, alongside the necessary metadata that is required to interpret the data. For example, in one embodiment, the combined data from a provider, a payer, and a community health program can be aggregated into a total outcomes data set, matched against a set of reference data, and output in the common information model 312. This data set can be further checked to ensure that the requesting user and/or application has sufficient access privileges (see, e.g., block 513 in Figure 4) and has the appropriate contracts / data use agreements / business associate agreements in place, if needed.

Following this, the data can sent out according to services calls of the API 314 (see, e.g., block 514 in Figure 4) in encrypted format (see, e.g., blocks 515 and 516 in Figure 4) to the requesting users / services. The users can now (see, e.g., blocks 517 and 518 in Figure 4) make use of a broader set of data to identify insights and make decisions. For example, a payer user who did not previously have access to outcomes-related data other than what was present on claims submitted may now have deeper insights into a patient's clinical experience based on a combination of data gathered during and after the experience (*e.g*., in community support).

### Example Use and Implementation of the Health Data System 100

Changes in the New Health Economy are forcing healthcare organizations to collaborate in order to deliver the comprehensive care that patients need to achieve target health outcomes. Once the health ecosystem necessary to address the needs of a patient population is proposed, the health data system 100 can act as an information interchange that facilities data/information exchange between members (*e.g*., data sources 201 and/or data consumers 207 as shown in Fig. 2). The health data system 100 can be an ecosystem enabler that identifies and connects the right network of health organizations (*e.g.*, data sources 201 and/or data consumers 207 as shown in Fig. 2) to improve consumer health among a targeted population. The health data system 100 can enable third-parties (*e.g*., data sources 201 and/or data consumers 207 as shown in Fig. 2) to transact in order to provide and/or consume the data and analytics necessary to achieve various desirable business objectives. By acting as an intelligent integration engine, the health data system 100 can facilitate connectivity across a marketplace to enable fluid exchange of data and services.

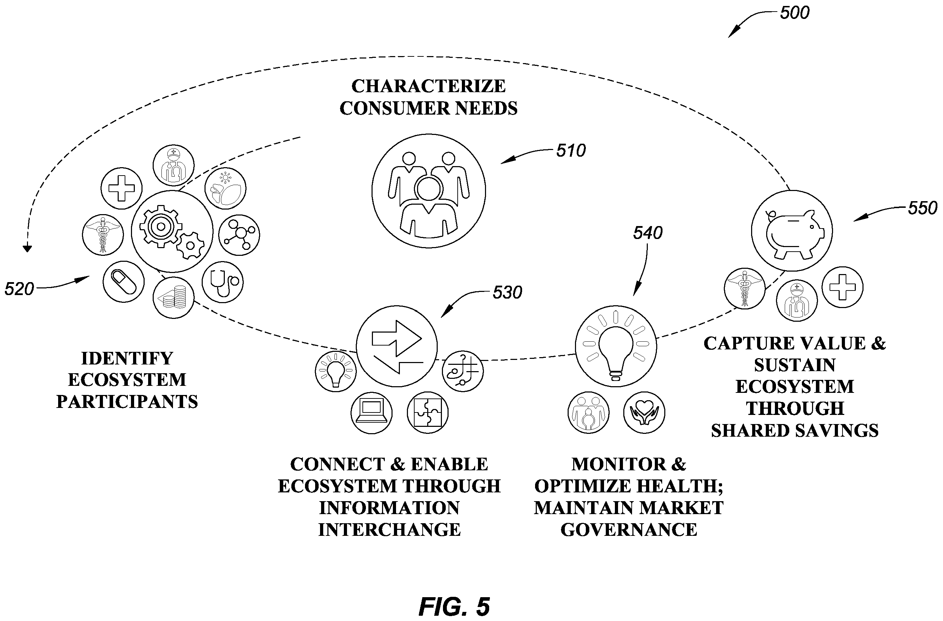

For example, the health data system 100 can identify and enable an ecosystem of individuals, organizations, and data-driven solutions in a program to improve disease management. In such an example use and implementation, the health data system 100 can connect such players as a mobile platform and a cognitive analytic solution to deliver continuous and seamless information to appropriate stakeholders (*e.g*., data sources 201 and/or data consumers 207 as shown in Fig. 2). Fig. 5 illustrates a novel method 500 to define a consumer health problem, create an ecosystem to resolve it, monitor the care delivery network to identify where health benefits are realized, and isolate the associated cost savings in order to redistribute them to both the risk-bearing entities and other ecosystem members. Specifically, the method 500 comprises defining targeted consumer/patient health challenges 510; identifying ecosystem participants and interactions required to address patient needs 520; using the health data system/information exchange 100 to facilitate ecosystem connectivity 530; optimizing interactions and implementing governance 540; and capturing value through consumer health improvements to sustain ecosystem through shared cost savings 550.

The health data system 100 can be configured to be and/or be a portion of a system that enables a comprehensive set of business and technology services, including the capability to intelligently locate distributed data, disseminate timely insights, and support a broad community of users across the care delivery network.

Such a health data system 100 can facilitate ecosystem interactions and act as a trusted broker of information exchange across stakeholders; connect to a variety of analytic applications that are able to access targeted data in a secure fashion in order to deliver intended outcomes such as improved education, health monitoring, improved health decision making, and disease prevention; provide access to data to facilitate ongoing reporting on outcomes of a disease treatment and prevention program including patient outcomes, care delivery effectiveness and efficiency, as well as value captured and savings achieved. In various embodiments, such a system can enable such reporting by providing access to the data through predefined dashboards, reports, score cards as well as ad-hoc analysis for a select number of stakeholders all based on appropriate permissions and agreements. Such reporting can also support tracking of funds flows. Additionally, such a system can be configured to ensure privacy and security of patient and stakeholder information through a defense-in-depth security model and develop market rules to manage interactions of members in the ecosystem.

To leverage the health data system 100 to improve care management (and other health care programs in other embodiments) a range of health stakeholders (*e.g*., data sources 201 and/or data consumers 207 as shown in Fig. 2) can be connected to share information. As discussed herein, the health data system 100 can be designed to provide access to data and analytics from across organizations in order to drive novel insights and care transformation.

Additionally, the health data system 100 engages ecosystem participants including, but not limited to, care delivery network entities (*e.g*., core providers, specialists, out-patient, hospital, clinics, urgent care, retail clinics that provide patient care); health management network (HMN) entities (*e.g*., risk-bearing entities (RBEs) such as government organizations, self-insured employers, and commercial payers; and non-RBEs that include employers (not self-insured), community centers, and patient advocates, which act as health promoters, educators, and the like). In some embodiments, such HMN groups can fund, subsidize, or otherwise incentivize target programs to optimize care delivery and reduce costs.

The health data system 100 can also be configured to engage commercial business network entities including, but not limited to, key business entities that should have a vested interest in driving treatment adherence and compliance given the impact of health spend on the American economy. Such entities can also serve as coordinators to drive health management by tailoring their products or delivery to the population and financially supporting community health initiatives.

Additionally, the health data system 100 can integrate fit-for-purpose technology solutions that meet the needs of the community, including a mobile patient engagement platform or a cognitive analytics expert system.

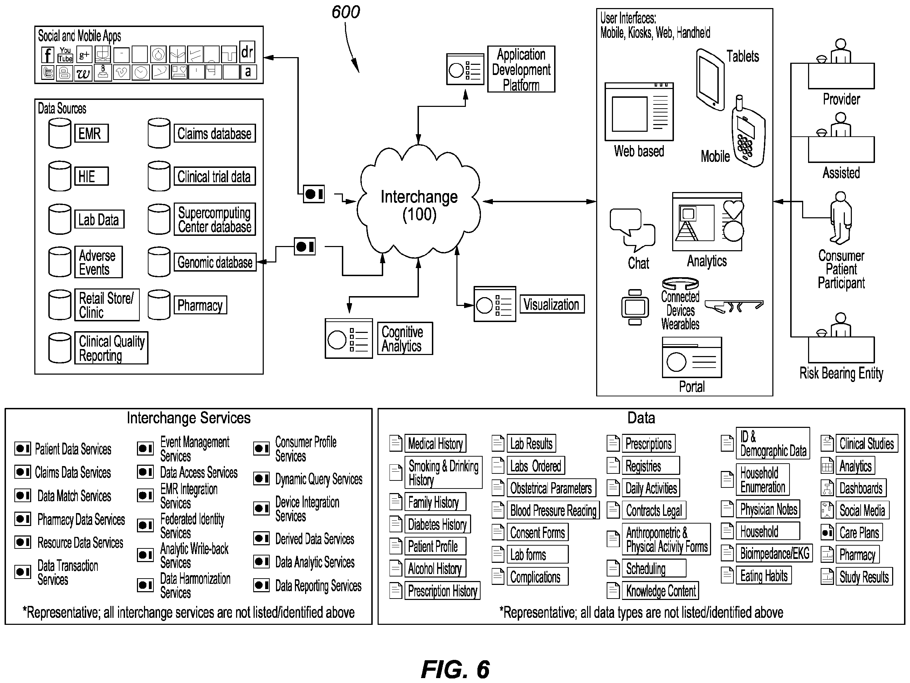

Since currently-available healthcare ecosystems are deficient, a healthcare ecosystem that provides for obtaining, storing and/or providing healthcare data can prove desirable and provide a basis for a wide range of applications as described in detail herein. This result can be achieved, according to one embodiment disclosed herein, by a healthcare ecosystem 600 as illustrated in Fig. 6. For example, Fig. 6 illustrates an embodiment of healthcare ecosystem 600, which involves an exchange of information from multiple sources delivered via web services (APIs). As shown in Fig. 6, the health data system 100 can serve as the data hub through which various ecosystem participants can connect and share data. For example, the health data system 100 can allow stakeholders in the ecosystem 600 to can gain access to data "in motion" (*e.g.,* the system 100 transits data only) or "at rest" *(e.g.,* the system 100 stores/persists the data in a repository as discussed herein).

In various embodiments, the health data system 100 can act as an interchange, where the system administrator does not own data or determine access rights/rules, but rather acts as a data steward to allow data owners (e.g., patients, clients and/or stakeholders) to dictate to which users and services the data will flow. The health data system 100 can enable the exchange of both regulated and unregulated data, and each entity in the ecosystem 600 can provide and receive data that is governed by relevant regulations and/or client-defined access rules (*e.g*., governed by the market rules 310 as shown in Fig. 2).

In various embodiments, the health data system 100 can enable data to be shared from a variety of sources, including consumer applications, medical devices, Electronic Health Records (EHRs), pharmacy records, and patient registries. Each of these data types can then feed into a wide range of applications supported by the health data system 100.

Data types in scope for various embodiments, such as a disease prevention and treatment program can include: provider primary clinical data (*e.g*., demographics, home medications, allergies, laboratory and pathology reports, transcribed records, and the like); provider secondary clinical data (*e.g*., natural language processing data, observation data, prognosis scores, analytics and statistical data, and the like); reference data for standardization (FHIR standard ontology, ISO 3166 codes, LOINC and SNOMED standard data sets, and the like); patient self-managed / self-reported data (e.g., glucose meter, wearables data, and the like); community advocate-related data (e.g., self-management program data, educational program data, and the like); financial / risk-bearing entity data (e.g., member plans, provider quality assessments, claims, and the like).

In various embodiments, the ecosystem 600 and/or health data system 100, can be configured to deliver the following services:

| **Functiontal Services** | **Description** |
|---|---|
| Core Information Interchange | Provides the technology platform to host data in a private, secure, scalable cloud, including: |
| | • **Hosting and Management:** Proprietary & 3^{rd} party data, analytics and applications |
| | • **Application Development Services:** Deliver APIs to enable development of new applications |
| | • **Always On Service/ Customer Support:** 7 x 24 real-time monitoring, support, and rapid response |
| | • **Security:** Layered security model creates safeguards to facilitate privacy and security protections and breach prevention that meets HITRUST control specifications |
| | • **Access Control:** Serving unique information access governance and policy requirements of ecosystem members |
| | • **Market Rules:** Manage interactions of members in the ecosystem through standard market terms and conditions, such as data access and use rules |
| | • **Monetization of Intellectual Property (Marketplace):** Enable 3^{rd} parties to transact in order to provide/consume the data and analytics necessary to achieve business objectives |
| Federated Identity Management | Linkage of a consumer's attributes defined by and stored across heterogeneous distinct source systems |
| Data Services | Aggregation and linking of evolving structured, unstructured and streaming data with lineage tracing |
| | ∘ **Data Quality:** Health data system 100 provides data quality services to help organizations QA their data to ensure it will be clean and standardized enough to deliver insights. The health data system 100 can also enforces data quality standards mandated by regulation, ecosystem covenants, or the like. |
| | ∘ **Patient Matching Service:** Health data system 100 can match multiple records from different sources (e.g., Hospital A and Hospital B) to the same master person record. |
| | ∘ **Data Integration Services:** From a source system to health data system 100 to avoid building many point to point integrations. Data flows are configurable and "plug and play" based on ecosystem member collaboration. |

As discussed herein, the health data system 100 architecture can be developed in a flexible manner to connect to a variety of data sources and services. For example, as illustrated in Fig. 2, such components can include:

| **Component** | **Definition** | **How Used & By Whom** |
|---|---|---|
| **Switch 311** | Can enable the compliant exchange, storage and access-controlled flow of information across stakeholders | The switch 311 can manage the flow of requests and the exchange of data across the platform 100, similar to a router, which as an analogy, manages the flow of data across a local area network (e.g., between a cable modem and various laptops and mobile devices). In some embodiments, users of the health data system 100, *(e.g.,* data sources 201 and/or data consumers 207) once authenticated to the platform 100, can have their data requests and exchanges facilitated by the switch 311. |
| **Distributed Agents 205** | Can enable connectivity of stakeholder data sources 201 to the health data system 100 market switch | A distributed agent 205 can be a program that connects to a stakeholder's local environment 201 to facilitate communication between that stakeholder 201 and the switch 311, which can be similar to an update utility that manages the flow of patches between a company (*e.g.*, Microsoft) and its customers. The agents 205 can be governed by a set of business rules 310 to enable adherence to stakeholder policies prior to receiving or sending any data to and from the health data system 100, and will be involved in the course of each data ingestion and transmission from a stakeholder 201 to the health data system 100. |
| **API(s) 314** | Can enable external users 207 to communicate and interact with the health data system 100 data and market applications through secure interfaces | Application programming interfaces (APIs) can be a standard means for developers of applications to share information about how to interoperate; for example, the ability to embed a Google Maps view or YouTube video in a website is facilitated via APIs. The health data system 100 APIs 314 can be a set of published standards that allow third party applications to authenticate to the health data system 100 and exchange data with the platform 100. The API(s) 314 can be used by various applications that interface with the health data system 100. |
| **Big Data Pathway/Sto rage Module 313** | Can enable storage, processing, and analysis of unstructured data | The Big Data Pathway/Storage Module 313 can be a technology component that enables the ingestion, management, and analysis of massive unstructured data sets (*e.g*., Twitter social media data, physician notes, etc.) in an efficient manner, using a variety of best-in-class packages such as Apache Flume, Cloudera (Apache) Hadoop, HP Vertica, and the like. These tools allow for the quick and cost-effective aggregation and searching through many terabytes and petabytes of data, and will be involved whenever big data analysis or analytics are desired. |
| **Health Level 7 (HL7) Transaction (not shown in** **Fig. 2****)** | Can enable interoperability with existing provider data services | The HL7 transaction set can specify a common language to be used when exchanging data with healthcare providers. In various embodiments, it is the *de facto* standard for clinical data transmission in the US, as featured in the HHS / CMS Meaningful Use guidelines, and is gaining adoption worldwide. The health data system 100 therefore supports HL7 transactions as a part of agent 205 and API 314 transmissions with healthcare providers or other stakeholders that require provider data. |

By leveraging the health data system 100 to enable a community-oriented approach to delivering care and managing chronic disease, various healthcare projects, have the potential to advance the shared mission of the national healthcare transformation effort and the Affordable Care Act (ACA). Specifically, the health data system 100 can have a beneficial impact on each of the ACA's "Triple Aims": providing more effective care and improve patient outcomes, through democratization of evidence-based care to where patients are while ensuring quality of care; providing care more efficiently and reduce the cost of care delivery, by triage and allocation enabled by integrated care delivery network with access to patient data for advanced analytics; and provide more consumer-centric care and improve patient experience, through uses of mobile and social platform to personalize care experience.

The described embodiments are susceptible to various modifications and alternative forms, and specific examples thereof have been shown by way of example in the drawings and are herein described in detail. It should be understood, however, that the described embodiments are not to be limited to the particular forms or methods disclosed, but to the contrary, the present disclosure is to cover all modifications, equivalents, and alternatives.

Additional applications can include:

### A. Improving chronic disease management by optimizing distribution of care across a network of care providers, retail clinics, and the home

Chronic disease management is a high cost, low return problem that plagues the healthcare industry. The problem spreads across multiple therapeutic areas such as diabetes, respiratory illness and cardio-vascular diseases. On an average two thirds of total healthcare spend is attributed to chronic disease management. Data driven chronic disease management has already shown tremendous potential in reducing cost and improving quality of life and the health data system 100 takes it to the next level through innovative interventions and consolidation of data.
1. System 100 is an integral part of a chronic disease management program, providing the core data management, ingestion, and application hosting services. System 100 provides delivery channel services that help caregivers/providers access the tools and data they need to provide care seamless across facilities, while patients will have similar access to tools and services they need to improve adherence to treatment regimens. Some key capabilities of the system 100 that enable the new ecosystem business model are listed below:
   **▪ The data gatherer:** System 100 provides the agent modules 205 that capture data from multiple sources, such as EMR (Electronic Medical Records), unstructured transactional and research data (through Natural Language Processing), financial data, patient reported information, and data streams from medical devices or fitness wearables. Data is captured from multiple organizations/sources and tagged accordingly.
      ∘ The data agent modules 205 scheduled to run at pre-defined intervals and/or can be executed on demand.
      ∘ Each data agent module 205 provides a collection of queries that run against data source system 201 and extract pre-defined set of data values.
      ∘ The data from data source 201 is transformed by the switch 311 using pre-defined algorithms and rule-sets and the transformed data values are stored in the common information model 312 either on persistent basis or in transitive mode
      ∘ For feedback purposes, the system 100 provides another set of templates/attributes/lists that are populated based on the outcome of the queries performed against the common info model 312 by the switch 311
      ∘ System 100 includes multiple out of the box agent modules 205. These agent modules 205 have been built to extract data from many 3^{rd} party EMR systems such as EPIC, CERNER, Centricity etc. and wearable devices such as BP and glucose monitors. The agent modules 205 are unique with respect to clinical and non-clinical attributes that are being extracted, as well as the optimized queries written for extraction. In addition, the agent modules 205 provide a unique plug-and-play capability wherein the need for configuration is minimized in case standard installations of transactional systems are being used by the client.
      ∘ System 100 contains a Natural Language Processing (NLP) engine that auto-tags unstructured data thereby reducing the effort of document-tagging by 20-30%.
      ∘ The platform allows the merging of structured, unstructured, and social data that is unique in context of healthcare industry.
   ▪ **Data consolidator:** System 100 performs significant amount of harmonization across the various ingested data sets, leveraging its unique transitive and preservative data model, and stores the cumulative analytical core data set as well as data received through feedback loop.
      ∘ Switch 311 maintains multiple code books that describe the translation of one coding scheme (terminology) to another.
      ∘ These code books are used to translate the inbound data's coding scheme to the common coding scheme established for the installation.
      ∘ For example one of the coding book explains the translation rules for diagnosis coded in ICD9 to SNOMED coding scheme
      ∘ In case no one-to-one coding map is available for a particular entry in a coding scheme, the switch module 311 leverages user-defined rules to resolve the conflict.
      ∘ The transitive and preservative scheme of data storage is controlled through a set of user-defined rules that distinguishes between the data to be stored and data to be passed along, at the time of execution
      ∘ For example there might be a rule that states that all data received from a particular source system needs to be stored and from another source system, needs to be pushed forward in a cumulative state.
      ∘ System 100 contains a data model, the common information model 312, that is patient-centric and includes multiple domains such as vitals, chronic, episodic, genomic, claims, social, behavioral etc. The common information model 312 is not only unique from attributes and relationship perspective but is also driven by user defined rules for storage perspective.
      ∘ The unique "transitive and preservative" concept deployed within system 100, allows the data administrator to control the flow of the data and its associated storage, through a set of editable rules. System 100 provides the data administrator control of what data should be stored persistently and what data should be flowed through on a temporal basis.
   ▪ **Data provider:** API 314 establishes an access layer for any application that needs to access the data managed in common info model 312. System 100 includes SDK (Software Development Kit) that contains API 314, predefined methods, and interface programs.
      ∘ API 314 can either be a collection of simple overloaded methods or complex web services
      ∘ A method or a service usually maintains the following structure: Collection/Data set API Name (parameterList) Where, Collection/Data_set is the return value provided by the API to the calling program/ system API_Name is the name that is used by the calling program to invoke the functionality delivered through the method/service Parameter List is a number of parameters that are passed by value or passed by reference to the being-called method in order to shape the outcome/return result from the method/service
      ∘ API definitions (signatures) are exposed through a pre-defined mechanism or through a pre-defined directory service, for example WSDL.
      ∘ APIs are called/invoked by the programs and systems that interact with the system 100
      ∘ The calling program passes the values for the exposed parameterList and the API return the response in form of either a collection or a dataset, based on the passed values.
      ∘ System 100 contains variety of services/methods, categorized under distinct API 314, that provide functionality to support the needs of the calling program/system. Some examples include, API 314 to return a consolidated medical record for a given patient, API 314 to expose results of an analytical query performed against a pre-determined cohort of patient population, API 314 to pass a secured message between two end users of the system 100 etc.
      ∘ The SDK (Software Development Kit) containing all the API 314 and other access mechanisms that are exposed by system 100 are unique to the system 100 wherein the signatures of the API 314, the overloaded input attributes and output collections have been designed and deployed specifically for system 100.
   ▪ **Security:** All data in the system 100, whether at rest or in motion, is protected through a sequence of security protocols in the security module 309. Security module 309 provides a role based dynamic, multi-form authentication and authorization. Data access is granted based on market rules 310 that establish authorizations based on data source system, organization source (and associated rules), and identities of the consuming user and application.
      ∘ Data at rest: All data stored in common information module 312 is encrypted using the latest encryption standards. The data placed in temporary areas, such as staging, is also encrypted. The encryption key is maintained at a distinct location in a double-blinded vault. Each user is assigned a set of roles that are maintained by the administrator of the platform. These roles provide the users to access certain screens, reports, modules, and code pieces. Even the users with same access grants may not see same data because of behind-the scene linkage between individual data items and authorized user-group.
      ∘ Data in motion: There are two primary areas for data in motion: 1) the data being retrieved from source systems, and 2) data being exposed to program/systems that need it through the API 314. Under both scenarios, the data flows through a preestablished secured tunnel. That secured tunnel is a combination of multiple rules sitting on top of baseline SSL protocol. Each layer of the platform (corresponding to ISO model) has its own distinct rule-set that augment the core SSL protocols.
      ∘ A 7-layer security and access control that is in line with the ISO model for software deployment. 1) Each of the ISO layer is independently handled through the security module 309, 2) the process/algorithm used to handle each ISO layer is proprietary to the system 100.
      ∘ The double-blinded vault to store encryption keys is a unique concept adopted from clinical trial industry
   ▪ **Server 120 is PHI Certified:** Server 120 is pre-configured to manage PHI (Protected Health Information) datasets through the use of latest and strictest available security protocols such as CS4096. Server 120 is HITRUST certified.
      - Server 120 is the only PHI carrying system built exclusively for Academic Medical Centers (AMCs) that is both HITRUST certified and deploys CS4096 encryption.
   ▪ **Analytical engine**: System 100 provides a core analytical platform that provides baseline analytical queries as well as propensity matched cohorts that allow for focused analysis out of vast data sets contained in the common information model 312 using the API 314.
      ∘ Data flows from the data sources 201 into the common information model 312.
      ∘ Data is harmonized by the switch module 311.
      ∘ The harmonized data is cumulated and/or de-normalized based on the analytical and/or transactional requirements for the data
      ∘ The de-normalized/cumulated data is stored in common information model 312
      ∘ Patient records are classified into various cohorts based on multiple factors such as age, gender, ethnicity, disease conditions, comorbidities, care program enrollment, medication types etc.
      ∘ Baseline inferences are drawn across these cohorts and stored as generic trends
      ∘ The generic trends are compared to 3^{rd} party benchmarks/standards and variances are stored in the platform
      ∘ When a query is executed using API 314, one or more patient cohorts are identified as target population for the query, depending upon the parameters passed by the query
      ∘ The associated patterns/trends are exposed to the calling program through API 314
      ∘ The variance between the trends and real life data is identified and quantified based on a pre-defined scale
      ∘ In addition the end-users are provided with the capability to over-ride the generated inferences
      ∘ The adjustment factors (based on either original patterns and real life data variances or end user over-rides) are fed back into common info model 312 and act as margin/probability adjustment factor for future iteration of similar query execution using API 314
      ∘ For cognitive queries, the trends are extrapolated into future and intangible factors such as typical behavioral reaction of a physician to a specific case, are superimposed on the baseline query response to provide almost real-world predictions
      ∘ The system 100 provides dynamic and self-learning analytics, *i.e.,* system 100 allows the end users to establish a reverse data flow from feedback perspective which in turn adjusts the boundary conditions, cut-off levels, percentage allocations, probability distribution and other mathematical equations to impact the next iteration of the execution of the analytical queries. The self-learning and self-adjusting behavior of the analytical responses based on transactional feedback gathered, is a unique to the system 100
   ▪ **Patient attribution:** System 100 precision triage API 314 takes a complete health profile of an individual and matches him or her to the best fit facility and health service based on health needs, provider specialties and capacity, insurance coverage, access/convenience, and other factors that influence value (quality, cost, experience).
      ∘ As data is ingested from the data sources 201 into the health server 120, each record is compared against a pre-defined, user-controllable set of attributes to link the record with variety of cohorts
      ∘ The cohort definition is pre-defined and is based on the ultimate functionality need of the system.
      ∘ For example for a chronic care management system, the user may want to break down all the patients into three categories: high-risk, medium-risk or borderline, and low-risk patients. For each of these classifications, the administrator may have defined a set of boundary conditions or rules. For example for a diabetes management program, the rule set could be as simple as the value of HBA1C; less than 5 classified as low-risk, 5-6 as borderline, and above 6 as high-risk.
      ∘ All the ingested data is pushed through these cohort definitions and each record is tagged against multiple cohorts thereby attributing the patients to various categories
      ∘ The patients, thus attributed into various cohorts, are further used by the analytical engine as target population for end-user queries.
      ∘ System 100 establishes relationships between patients and various care delivery aspects. These care delivery aspects could vary from incentive program enrollments, care coordination, pre-section for trials etc. The linkage allows the patients to be put under various cohorts thereby simplifying the later query execution as well as improving performance tremendously.
   ▪ **Consumer engagement:** The consumer engagement API 314 implements a patient portal with customized content based on an individual's health profile, including geo-located and personalized incentives to encourage healthy behavior, game-based tools that engage consumers to manage their health and wellness, and direct connectivity to their healthcare providers.
      ∘ The patient portal is an integrated gateway to be used by the patient to interact with system 100
      ∘ Individual patients access the portal through a secured mechanism that deploys multi-form authentication that could contain biometric authentication also, such as finger-print and/or retinal scan
      ∘ Once in the portal, the patient is presented with a dashboard of their cumulative health, including a proprietary healthScore
      ∘ The healthScore is created based on patient's medical condition through the use of proprietary algorithms that leverage variety of factors such as patient's demographic data, vitals, concomitant meds being consumed, comorbid disease conditions, prior history, family background, and multiple other factors.
      ∘ The portal also offers multiple choices to the patients including ability to interact with the caregivers, scheduling appointments, refilling prescriptions, validating and adjusting their care plans, learning through provided/recommended educational material etc
      ∘ The patient portal is unique from the integration and harmonization perspective, *i.e*., it is a single gateway that allows the patients to see all their authorized data at one spot. The data includes patient's medical data, lab results, radiology images and reports, prescription data, their wearable device data as well as wave forms (streaming) from devices such as EKG. All these devices and system of records may use differing terminologies. System 100 translates them into a common language and terminology.
      ∘ Another unique feature of the platform is that it renders the same information through same portal across multiple delivery channels such as smart devices, laptops, phones etc.
   ▪ **Clinical Decision Support System:** The system 100 provider care delivery service includes a provider portal with customized applications, patient clinical and pathology data, clinical resources like comparator data from public resources. These capabilities will provide a longitudinal account of patient data to improve cooperation across caregivers and coordination across care facilities.
      ∘ The CDSS module of the platform can be rendered one of the two ways: 1) as a standalone application, and 2) as an integrated extension of existing transactional system
      ∘ Under the second scenario, the CDSS module is usually invoked through the click of a button within the core transactional application being used by the end user, such as an EMR system for a caregiver.
      ∘ The patient Id for the patient under investigation, is passed to the CDSS module
      ∘ The CDSS module retrieves and harmonizes the entire available medical history for the passed patient Id.
      ∘ The CDSS module harmonizes the patient medical history against the common terminology and standards being executed by the transactional system and presents the longitudinal record
      ∘ In addition the CDSS module can be configured to provide recommendations associated with the care plan for the therapeutic pathway under consideration. These recommendations are based on the patient's individual traits as well as industry benchmarks and best practices
      ∘ From a CDSS perspective, the system 100 is driven by a set of behind-the-scene rules that have been devised by clinicians and are used to correlate a patient's medical condition to one or more pre-defined therapeutic pathways in order to establish the future care plan.
      ∘ The CDSS module leverages multiple industry benchmarks and best practices in order to make recommendations for fine-tuning future care plans
   ▪ **Outcome rate**: 10-20% health outcomes improvement as measured by core clinical parameter associated with the disease condition. For example 10-20% improvement on HBA1C measure for diabetes or PFT score for asthma/COPD
   ▪ **Cost reduction**: 15-25% cost reduction as measured against TCM (Total cost of Management) for the disease condition. This cost reduction is measured through reduction in high-cost activities (such as trips to ER) calculated across multiple intervention cohorts
   ▪ **Compliance rate**: 20-30% improvement in compliance rate as measured not by the orders filled but orders consumed by patient.
   ▪ **Access improvement**: 20-25% improvement as measured through a weighted combination of various factors, such as reduction in time to schedule appointments, reduction in days for the appointment, reduction in days to access lab results etc.

### B. Standardizing cancer treatment to reduce care variability for an integrated cancer care delivery network

1. Over one trillion dollars in U.S. health care expenditures are considered wasteful, driven by inefficiency in care delivery, suboptimal care, and inadequate access. Cancer care is one of the most constrained due to insufficient access to expert knowledge to the general oncology practitioner, lack of evidence-based tools and practices to minimize treatment variations, and an inability to engage with the patient to efficiently manage their disease. Current reimbursement models do not incentivize investments in long term wellness, instead focusing on treatment volume. These factors create gaps in treatment quality and increase costs. A new health system is needed to deliver enduring changes enabled by innovative data-driven solutions that demonstrate improved outcomes and cost savings at-scale. The MD Anderson Cancer Center (MDACC) Network Democratization project is one such initiative, committed to accelerating clinical care for cancer patients by improving outcomes through evidence-based and experience-informed clinical decisions.
2. The network democratization initiative focuses on the following broad level activities
   ▪ Creating of an integrated provider partner ecosystem that consists of Providers (to manage patients), Risk Bearing Entities (that pay for the care), and Technology partners (to enable allocation of the right care, facilitate care continuity, and improve care quality)
   ▪ Securely ingesting data from multiple sources (such as EMR systems) for cancer patients. Standardizing clinical data into a common format and terminology to create a curated disease summary, identifying treatment recommendations for demographically similar cohorts based on expert knowledge, enabling expert virtual consults for diagnosis and staging confirmation, and providing care pathway advisories to manage the disease
   ▪ Creating avenues for targeted interventions through education and cancer screening services
   ▪ Shifting preventative screening and early diagnosis to the community providers (60%), personalized care supported by expert advisory and live expert consultation (30%), and facilitating rapid referrals if the expertise is not available at the local network partner (10%)
   ▪ Making available consistent and consolidated information to all involved stakeholders (physician, support personnel, patient) through web and mobile solutions
   ▪ Leveraging a longitudinal view of the patient's cancer and medical history data to fine-tune treatment recommendations
   ▪ Comparing cost and outcomes for intervention programs against nationally and/or internationally established benchmarks, to further tune interventions for the right outcomes
3. System 100 is an integral part of network democratization, providing core data management, data ingestion/standardization, and analytics hosting.
   ▪ **External Data Connectors and Services**: The agent modules 205 capture data from multiple sources, such as EMR (Electronic Medical Records) systems resident at a network provider partner, unstructured clinical data (used to ingest secondary information about the patient), and patient self-reported information (such as a cancer survey completed during a hospital onboarding event)
      ∘ The agent modules 205 can be scheduled to run at pre-defined intervals and/or can be executed on demand.
      ∘ Each agent module 205 includes queries that run against the source 201 to extract pre-defined set of data values.
      ∘ These data attributes can be controlled through templates that come out of the box with system 100. These templates can be modified at installation time or during execution by an administrator of system 100.
      ∘ The extracted data is transformed using pre-defined algorithms and rule-sets and the transformed data values are stored in 312
      ∘ System 100 includes multiple out of the box agent modules 205 that are proprietary to the platform. These agent modules 205 have been built to extract data from both custom and 3^{rd} party EMR systems such as EPIC, CERNER, etc. and wearable devices such as BP and glucose monitors. The agent modules 205 are unique with respect to clinical attributes that are being extracted, the proprietary two-step process being used for extraction and transformation, as well as the optimized queries written for extraction. In addition, the agent modules 205 provide a unique plug-and-play capability wherein the need for configuration is minimized in case standard installations of transactional systems are being used by the client.
      ∘ The system 100 allows the merging of structured and unstructured data that is unique in context of care delivery
   ▪ **Data Management:** System 100 standardizes data across the various data sets to a variety of clinical data exchange standards, leveraging its unique patient-centric common information model, and persists primary clinical data, as well as derived secondary information (*e.g*., prognostic scores) collected from clinical notes (*e.g.*, secondary clinical diagnoses)
      ∘ System 100 maintains multiple coding dictionaries that describe the translation of one coding scheme (terminology) to another.
      ∘ These coding dictionaries are used to translate the terms in the data from data source 201 into a set of standard terms used in system 100.
      ∘ For example one of the coding dictionaries contains translation rules for clinical diagnoses coded in ICD9 to SNOMED coding scheme. Another example translates lab data into the LOINC coding scheme
      ∘ In case no one-to-one coding map is available for a particular entry in a coding scheme, system 100 leverages user-defined rules to resolve the conflict. System 100 persists both the as-is and translated values
      ∘ The transitive and preservative scheme of data storage is controlled through a set of user-defined rules in 310 that distinguish between the data to be persisted in 312 and data to be obtained dynamically from 201 at the time of a request for this data by 130
      ∘ For example there might be a rule that states that all data received from a particular source system needs to be persisted in 312 and from another source system, needs to be obtained from 201 on demand.
      ∘ System 100 contains a data model, common information model 312 that is patient-centric and includes multiple domains such as demographics, home medications, allergies, vitals, observations, labs, and clinical document relevant to cancer care. 312 is not only unique from attributes and relationship perspective but is also driven by user defined rules for storage perspective.
      ∘ The unique "transitive and preservative" concept in system 100, allows data source owner and / or administrator of system 100 to control the amount of data from data source 201 that is persisted in 312 by an administrator of system 100.
   ▪ **Security:** System 100 uses a role based dynamic, multi-form authentication and authorization protocol 310. Data access is granted based on rules that establish authorization based on data source, and identities of a consuming user (*e.g.,* a Lung physician at a Community hospital) and consuming application (*e.g*., Oncology Expert Advisor analytic tool). System 100 is pre-configured to manage PHI (Protected Health Information) datasets through the use of latest and strictest available security protocols such as CS4096. In addition, the system 100 is HITRUST certified
   ▪ **Access, Discovery, and Analytics:** System 100 contains over 200 API 314 purpose-built for network democratization, to establish an information delivery and access layer for consuming analytics tools.
      ∘ API 314 can either be a collection of simple overloaded methods or complex web services
      ∘ A method or a service usually maintains the following structure:
         Collection/Data set API_Name (parameterList) Where, Collection/Data_set is the return value provided by the API to the calling program/system
         API_Name is the name that is used by the calling program to invoke the functionality delivered through the method/service
         Parameter List is a number of parameters that are passed by value or passed by reference to the being-called method in order to shape the outcome/return result from the method/service
      ∘ API definitions (signatures) are exposed through a pre-defined mechanism or through a pre-defined directory service, for example WSDL.
      ∘ API 314 are called/invoked by the user devices / services 130 that interact with system 100.
      ∘ The calling program passes the values for the exposed parameterList and the API return the response in form of either a collection or a dataset, based on the passed values.
      ∘ System 100 contains variety of services/methods, categorized under distinct API 314, that provide functionality to support the needs of the calling program/system. Some examples include, API to return a consolidated medical record for a given patient, API to expose results of an analytical query performed against a pre-determined cohort of cancer patients, or an API to pass a secured message between two network partners using system 100
   ▪ **Key Outcomes**: Reduced variability in cancer treatment, improved diagnosis and staging accuracy, and better adherence to care pathways leading to reduced unnecessary ER/hospital visits and empowered patients with education, screening, and adherence tools
   ▪ **Cost Reduction:** Lung cancer creates over 220K new patients in the US each year, with an estimated $8 - $10B spend on treatment of non-small cell lung cancers (NSCLC) annually (treatments are 50% more expensive for patients over 65 years of age). The outcomes indicated above represent a 16% savings opportunity in the overall cost of lung cancer treatment, translating to a $180 - $240M reduction in cost of care for NSCLC (or 85% of new lung cancer cases diagnosed each year)
   ▪ Breast cancer creates over 230K new patients in the US each year, with an estimated $16 - $17B spend on treatment (treatment at stage 3 and 4 of breast cancer can cost up to five times more than stages 1 and 2). The outcomes indicated above represent an 8 - 12% savings opportunity in the overall cost of breast cancer treatment, translating to a $192 - $204M reduction in cost of care assuming a 5 - 10% patient engagement rate (of the overall population)

### C. Increasing therapy adherence to improve chronic disease outcomes for pharma companies

1. Medication non-adherence represents a $300B challenge to the pharma industry - leading to 125K deaths each year (4^{th} leading cause of death, 1^{st} for accidental deaths), causing 10 - 20% of hospital or nursing home admissions, and $188B in biopharma revenue loss. Today's adherence programs solve for this challenge through limited patient communication via traditional channels (mail/email/call), are not based on real-time data about dosing behavior, limit health care professional's (HCP) ability to link adherence to symptom improvements, and are unable to continuously improve efficacy of messages to patients. In response, Pharmas are launching digital and analytics enabled solutions to personalize interventions and demonstrate improved outcomes through increased patient engagement.
   System 100 plays a unique role in helping pharma companies play a role in actively monitoring patient symptoms, stay on recommended therapy regimens, and reinforce healthy behaviors (*e.g*., getting a refill, seeing a clinician). System 100 facilitates secure continuous ingestion, processing, and monitoring of informed patient consent, combines sensitive information (PHI) related to dosage, combining it with other patient information such as insurance claims, clinical records, and self-reported symptoms. This data is used by analytic algorithms to draw inferences such as propensity scores, adherence patterns, and outcome metrics to stratify patients by risk and recommend interventions; patient and provider tools then communicate with the patient or HCP with targeted interventional and/or educational content to improve adherence behaviors and effect outcomes.
   ▪ Creating an integrated partner ecosystem comprising Health Care Professionals (HCPs that treat patients), Risk Bearing Entities (insurers that facilitate Medical and/or Pharmacy claim processing), Patients, and Technology partners (device partners to signal adherence data, partners to enable personalized mobile communication, analytics partners to generate individual or cohort-level insights)
   ▪ Securely ingesting and harmonizing data from multiple sources into a common format to stratify at-risk individuals, understand what interventions work for whom and via what channel, and customize messages for therapy-related engagement
   ▪ Facilitating targeted interventions (messages, in-app alerts, educational content, dashboards) for the Patient and/or HCP to supplement existing reminders from specialty pharmacies
   ▪ Conducting post market studies to demonstrate impacts of improved adherence
2. System 100 is an integral part of adherence solutions, providing secure data collection, standardization, and analytics hosting.
   ▪ **Analytical engine:** System 100 provides a core analytical platform, implemented as a of services provided by the API 314, that provides baseline analytical queries as well as propensity matched cohorts that allow for focused analysis out of heterogeneous data sets
      ∘ Data flows from source systems into system 100.
      ∘ Through the data gatherer and data consolidator roles, the data is ingested and harmonized
      ∘ The harmonized data is cumulated and/or de-normalized based on the analytical and/or transactional requirements for the data
      ∘ The de-normalized/cumulated data can be stored in specific data marts for targeted analysis
      ∘ Patient records are classified into various cohorts based on multiple factors such as age, gender, ethnicity, disease conditions, comorbidities, care program enrollment, medication types etc.
      ∘ Baseline inferences can be drawn across these cohorts and stored as generic trends that can be used to customize and recommend interventions
      ∘ The generic trends are compared to 3^{rd} party benchmarks/standards and variances are stored in the platform
      ∘ When a query is executed, one or more patient cohorts are identified as target population for the query, depending upon the parameters passed by the query
      ∘ The associated patterns/trends are exposed to the calling program through API 314
      ∘ The variance between the trends and real life data is identified and quantified based on a pre-defined scale
      ∘ In addition the end-users are provided with the capability to over-ride the generated inferences
      ∘ The adjustment factors (based on either original patterns and real life data variances or end user over-rides) are fed back into system 100 platform and act as margin/probability adjustment factor for future iteration of similar query execution
      ∘ For cognitive queries, the trends are extrapolated into future and intangible factors such as typical behavioral reaction of a physician to a specific case, are superimposed on the baseline query response to provide predictions
      ∘ System 100 offer dynamic and self-learning analytics, *i.e.*, system 100 allows the end users to establish a reverse data flow from feedback perspective which in turn adjusts the boundary conditions, cut-off levels, percentage allocations, probability distribution and other mathematical equations within the analytical engine in order to impact the next iteration of the execution of the analytical queries. The self-learning and self-adjusting behavior of the analytical responses based on transactional feedback gathered, is a unique preposition of the system 100.
   ▪ **Information delivery gateway**: The system 100 consumer engagement service includes a patient portal with customized content based on an individual's health profile, including geo-located and personalized incentives to encourage healthy behavior, game-based tools that engage consumers to manage their health and wellness, and direct connectivity to their healthcare professional (HCP)
      ∘ The system 100 patient portal is unique from the integration and harmonization perspective, *i.e*., it is a single gateway that allows the patients to see all their authorized data at one spot. The data includes patient's medical data, lab results, radiology images and reports, prescription data, their wearable device data as well as wave forms (streaming) from devices such as EKG. All these devices and system of records may use differing terminologies.
      ∘ Another unique feature of the platform is that it renders the same information through same portal across multiple delivery channels such as smart devices, laptops, phones etc.
   ▪ **Facility for end-to-end sensitive patient data management**: System 100 uses a role based dynamic, multi-form authentication and authorization protocol 309. Data access is granted based on rules that establish authorization based on data source, and identities of a user (*e.g*., Patient, Pharma, HCP user), system (*e.g.*, EMR system providing patient clinical data). System 100 is pre-configured to manage PHI (Protected Health Information) datasets through the use of latest and strictest available security protocols such as CS4096. In addition, the system 100 is HITRUST certified

### D. Improving financial and spend performance using advanced analytics and multi-data collection leading to activity based costing

**1.** Large scale institutes (Academic as well as pure play commercial) with multiple facilities spread across geographically, usually are forced to manage a diverse and complicated supply chain. The "spend" is usually highly distributed with a lack of centralized coordination, leading to significant redundancy as well as wastage. The critical challenges faced under such situations range from under-utilization of inventories at one facility whereas same inventory is in short supply at another facility, identification of best vendors for local quick-lead supplies as well as centralized long-lead needs, Economic Order Quantity and reorder level identification etc. These supply chains are also encumbered by the fact that usually they are a network of multiple locally operated ordering and procurement systems, thereby highly unlikely to track "spend" across an individual patient's life cycle.
   There is an incremental trend that the spend analytics is moving towards and that is to establish a solid platform to calculate and manage 'activity based costing' in order to support the latest healthcare paradigm of bundled payments. It is critical for any large institution to understand the details of cost broken down by each involved activity under a large scale procedure (such as hip replacement) for them to be able to logically accept an offered bundled payment for the procedure by the RBEs.
**2.** Any well-tuned centralized spend analysis system focuses on following broad level activities
   ▪ Creation of an integrated ecosystem that consists of suppliers (local as well as centralized), inter-linked facilities that service the patients and a centralized governance authority to manage the flow of data across all stakeholders and to manage the global rules that govern procurement.
   ▪ Ingestion of data from multiple local ordering and procurement systems
   ▪ Ingestion of data from local clinical and operational systems such as EMRs, scheduling systems, pharmacy systems, financial systems, and capacity management systems.
   ▪ Ingestion of data from third party sources that provide benchmarks and best practices across the country.
   ▪ Harmonization of "spend" and clinical data to generate a common script for the centralized authority.
   ▪ Spend data categorization across multiple factors such as type of material/services, geographical demand and availability, clinical necessity and urgency, capital or non-capital needs etc.
   ▪ Tying the spend data to clinical and/or therapeutic pathways in order to generate activity based cost for each sub-activity of a large procedure. This information is critical for the newer payment models coming up in the health industry, such as payment for outcomes rather than payments for service.
   ▪ Making available consistent and consolidated information to all involved stakeholders (local facilities, suppliers, centralized authority etc) through various delivery channels
   ▪ Establishing spend patterns, trends, lead times, replenishments etc based on harmonized spend data
   ▪ Comparison of spend trends against 3^{rd} party benchmarks to identify the variances and associated mitigation approaches
   ▪ Ability to establish rules (user controlled) to cover for cost versus clinical urgency requirements
   ▪ Ability to establish rules to manage and support local variances
   ▪ Ability to receive feedback and incorporate it in order to fine-tune future transactions
   ▪ **PHI Certified System 100**: System 100 is pre-configured to manage PHI (Protected Health Information) datasets through the use of latest and strictest available security protocols such as CS4096. In addition the system 100 is HITRUST certified.
      - **Uniqueness**: System 100 is the only PHI carrying platform built exclusively for Academic Medical Centers (AMCs) that is both HITRUST certified and deploys CS4096 encryption.
   ▪ The system 100 patient portal is unique from the integration and harmonization perspective, i.e., it is a single gateway that allows the patients to see all their authorized data at one spot. The data includes patient's medical data, lab results, radiology images and reports, prescription data, their wearable device data as well as wave forms (streaming) from devices such as EKG. All these devices and system of records may use differing terminologies. System 100 translates them into a common language and terminology.
      ∘ Another unique feature of the platform is that it renders the same information through same portal across multiple delivery channels such as smart devices, laptops, phones etc.
   ▪ **Activity based costing engine:** System 100 ties financial data with clinical data to establish spend patterns against each sub-activity of a large therapeutic procedure. For example system 100 can break down spend against pre-op, day-of-op, and post-op care activities for a typical hip replacement procedure that is neither chronic nor acute care but instead spreads across a pre-defined time-frame.
      ∘ System 100 contains activity level breakdown of multiple therapeutic pathways for a variety of disease conditions. These pathways are neither chronic not episodic. Instead they focus on a period of time wherein patient was treated for a particular disease condition or abnormality.
      ∘ For example, there is a detailed breakdown of all pre-op, operational, and post-op activities associated with hip and knee replacement process.
      ∘ Post financial-data-ingestion, the platform tags the transactional data against these activities that cumulate to formulate the complete process
      ∘ This categorization of financial transactions is cumulated across multiple patients and multiple facilities
      ∘ Analytics is performed against these cumulative numbers to establish factors such as average cost per activity for a particular therapeutic pathway, best facility to perform a certain activity based on cost as well as outcome rates etc.
      ∘ These anal
   ▪ **Contract support engine:** System 100 leverages the trends established through activity based costing to provide analytical support for providers as well as RBEs in order to establish contract terms for newer payment models such as value based payments and/or bundled payments.
      ∘ As an activity based costing engine, system 100 breaks down certain therapeutic pathways into sub activities.
      ∘ For example there is a clear list of pre-op, day-of-op, and post-op activities that have been defined for a hip replacement procedure. These activities could span a considerable amount of time but are not considered chronic conditions.
      ∘ It is imperative to understand the cost associated with each of these sub-activities in order to establish contracts under new payment models for the healthcare industry. Models such as fee for value rather than fee for service or bundled payment contracts.
      ∘ System 100 leverages baseline benchmarks created under activity-based-costing module, for each sub-activity, considering variety of factors such as patient-mix, geographical and environmental correlations, and health outcomes, and helps the financial administrators in figuring out what is the optimum contract terms that they can establish with risk bearing entities, for a given therapeutic pathway.
   ▪ **Capacity Utilization:** 10-15% improvement on capacity utilization based on centralized procurement based on system 100 suggested EOQ and replenishment levels. This is also impacted by the geographical distribution of the facilities as well as extent of centralized procurement.
   ▪ **Contract Efficiency:** 10-12% higher accuracy in terms of matching actual spend against the offered reimbursement by RBEs. These matrices are also impacted by the volume of such bundled payments as well as clearly identified sub-activities within a therapeutic pathway.
   ▪ **Patient-mix based capacity adjustment:** 15-20% improvement in capacity utilization as measured against the patient-mix at a facility. System 100 provides recommendations, based on patient-mix as well as volumes, regarding the optimum capacity for each facility, leading to the aforementioned reduction in wastage and/or over-stocking
   ▪ **Administrative cost efficiency:** 12-14% cost efficiency (reduction in overall spend system cost against system 100 cost) is a typical ROI that can be achieved through system 100

### E. Accelerating translational cancer research at a comprehensive cancer care center

**1.** Cancer is the second leading cause of death in the USA with almost 600,000 Americans succumbing annually and generating $175 billion in healthcare system cost. As a result of decades of investment in cancer research, the life expectancy for many of the 1.6 million Americans diagnosed with cancer every year is steadily increasing. However, much remains to be done as there are still relatively few true cancer cures, and some cancer types have seen little improvement in mortality over the last few decades. Cancer is now recognized as essentially a disease of the genome. DNA damage leads to uncontrolled cell division and often rapid cellular evolution which enables cancerous populations to develop resistance to therapy. There is a big push to translate the latest breakthroughs in the genomic sciences to cancer care delivery to create personalized medicine approaches tailored to each patient's particular cancer genome. This typically involves integrating large scale genomic and clinical data on the order of many gigabytes per patients and petabytes across even relatively small cancer cohorts.
**2.** Translational cancer research typically involves:
   ▪ Acquiring vast amounts of 'omic data on each patient including genomic, epigenomic, RNA sequences, and proteomics data from both the germline and the cancer as it evolves over time.
   ▪ This patient-level 'omics data must be combined with other laboratory assays and rich clinical data from the patient's medical record including diagnoses; surgical, radiation, and chemotherapy; responses to treatment, and, ultimately, outcomes.
   ▪ Once these data are integrated for each patient, cancer researchers query the data looking for gene mutations and other biomarkers that predict prognosis and response to therapy.
   ▪ These incites are useful for generation hypotheses about cancer biology, new treatment modalities, and cancer population health as well as to design clinical trials.
   ▪ In some cases, this integrated data can also inform actually clinical therapeutic chooses for individual patients, i.e. personalized medicine
3. System 100 is an integral part of a translation cancer research program, providing the core data management, ingestion, and application hosting services. System 100 is also provides delivery channel services that help cancer researchers access the tools and data they need to drive their research in the lab, at the bedside, and across patient populations. Some key system 100 capabilities that enable the new ecosystem business model are listed below:

### F. Increasing therapy adherence to improve chronic disease outcomes for pharma companies

The Information Interchange helps leading pharma companies play a unique role in using data and analytics to provide tools directly to patients and their health care provider teams that help them monitor symptoms, stay on the recommended therapy regimen, and reinforce healthy behaviors (*e.g*., getting a refill, seeing a clinician). The Interchange facilitates secure continuous ingestion, processing, and monitoring of informed patient consents, and combines sensitive information (PHI) related to dosage from wearables (*e.g*., Fitbit, Apple Watch), drug injectors, insurance claims, clinical profiles, and self-reported health and activity from patient mobile applications.

The sensitive information is then used by analytics algorithms for inferences (*e.g*., propensity scores, adherence patterns, outcomes metrics) to stratify patients by risk, and to recommend interventions based on predicted behaviors; patient and provider-specific tools then inform the patient and their clinician with targeted interventional and informational content.

### Improving Product Quality and Safety of Imported Drugs

The system 100 can serve as an information interchange that will enable the pharmaceutical industry to satisfy the federal expectations set forth in Title VII of the Food and Drug Administration Safety and Innovation Act (FDASIA) (Pub. L. 112-144). Title VII provides the FDA new authorities to help ensure the safety, effectiveness and quality of drugs in the United States. For example, the information interchange could help with the following subset of the 18 sections of Title VII:
1) Sec 705: Risk-based Inspection Frequency. Off shore drug manufacturers will need to submit data periodically to the FDA in order to determine a risk-based schedule of inspections of off-shore facilities with the finite number of inspectors available. Thus, the interchange will enable manufacturers to integrate product, facility, raw material, and operator data readily for evaluation by quality system analytics. This ability to produce product quality data periodically not only enables manufacturers to comply with this section of Title VII, but also enables review of their quality systems and to take proactive action prior to an inspection.
2) Sec 710: Exchange of Information. FDA may enter into reciprocal agreements to furnish certain trade secret information to foreign governments that have the authority and demonstrated ability to protect trade secret information. The HITRUST certified information interchange is uniquely positioned to support handling of trade secrets and other proprietary aspects of product quality or compound composition data.
3) Sec 711: Enhancing the Safety and Quality of the Drug Supply. The information interchange can improve the oversight and control that manufacturers demonstrate over the manufacture of quality drugs, including raw materials and finished products. See diagram below of quality systems data that interchange may monitor and integrate to support this reporting requirement.

## Claims

1. A health data system (100) for delivering patient-centric and value-based care, comprising:
a health data server (120);
two or more health data sources (110) in communication with the health data server over a secured network (140), wherein said health data sources each have a set of polling permissions and wherein said data sources comprise body monitor devices;
one or more agent modules (205) of the health data server that poll health data from the data sources at a designated frequency based on a set of identifiers and the set of polling permissions;
a first switch module (311) for providing the polled health data into a common information model (312), the common information model being defined by at least one patient record, each patient record having a plurality of attributes, and wherein the first switch module is configured to match the polled health data from the two or more data sources to the at least one patient record using a total match score, the total match score based on a sum of two or more weighted scores applied to the attributes, wherein the attributes of the polled health data are assigned predetermined weights determined based on the likelihood of change throughout a patient's life and, upon matching, the polled health data is tagged with a unique identifier for the patient in the health data system; and
one or more interface modules for gaining access to the common information model based on a set of access permissions.

2. The health data system of claim 1, wherein the common information model is stored as a distributed database and wherein the one or more attributes optionally define at least one of clinical health data, laboratory data, remote monitoring data, biometrics, wearables, social media data, self-reported data, mobile application data, and device instrumentation.

3. The health data system of claim 1 or claim 2, wherein said first switch module (311) augments the common information model with a new attribute when the polled health data does not map into the one or more attributes.

4. The health data system of any one of claims 1-3, wherein said first switch module (311) at least one of filters the polled health data prior to providing the polled health data into the common information model based on storage permissions, the storage permissions optionally being provided by at least one of the health data server and the one or more health data sources, and controls network connections over the secured network.

5. The health data system of any one of claims 1-4, wherein the designated frequency is set by at least one of the health data server (120) and the one or more health data sources (110).

6. A method for delivering patient-centric and value-based care, the method comprising:
polling two or more health data sources (110) for health data via one or more agent modules (205) of a health data server (120), wherein each of the one or more health data sources have a set of polling permissions and wherein the two or more health data sources (110) are in communication with the health data server over a secured network (140), and wherein said polling is performed at a designated frequency based on a set of identifiers and the set of polling permissions; and wherein said data sources comprise body monitor devices;
populating a common information model with the polled health data via a first switch module (311), the common information model being defined by at least one patient record, each patient record having one or more attributes and wherein the method includes matching the polled health data from the two or more data sources to the at least one patient record using a total match score, the total match score based on a sum of two or more weighted scores applied to the attributes, wherein the attributes of the polled health data are assigned predetermined weights determined based on the likelihood of change throughout a patient's life and, upon matching, the polled health data is tagged with a unique identifier for the patient in the health data system; and
providing access to the common information model via one or more interface modules based on a set of access permissions.

7. The method of claim 6, wherein said populating the common information model comprises populating a distributed database optionally with the one or more attributes selected from at least one of clinical health data, laboratory data, remote monitoring data, biometrics, wearables, social media data, self-reported data, mobile application data, and device instrumentation.

8. The method of claim 6 or claim 7, further comprising augmenting the common information model with a new attribute when the polled health data does not map into the one or more attributes via the first switch module.

9. The method of any one of claims 6-8, further comprising filtering the polled health data based on a set of storage permissions prior to said populating.

10. The method of any one of claims 6-9, wherein the set of storage permissions are provided by at least one of the health data server and the one or more health data sources.

11. The method of any one of claims 6-10, wherein said polling frequency is set by at least one of the health data server and the one or more health data sources.

## Patentansprüche

1. Gesundheitsdatensystem (100) zum Liefern von patientenzentrierter und wertbasierter Versorgung, das Folgendes umfasst:
einen Gesundheitsdatenserver (120);
zwei oder mehr Gesundheitsdatenquellen (110), die sich über ein gesichertes Netz (140) in Kommunikation mit dem Gesundheitsdatenserver befinden, wobei die Gesundheitsdatenquellen jeweils einen Satz von Abfrageberechtigungen aufweisen und wobei die Datenquellen Körperüberwachungsvorrichtungen umfassen;
ein oder mehrere Agentenmodule (205) des Gesundheitsdatenservers, die Gesundheitsdaten von den Datenquellen in einer vorgesehenen Frequenz basierend auf einem Satz von Kennzeichen und dem Satz von Abfrageberechtigungen abfragen;
ein erstes Schaltmodul (311) zum Bereitstellen der abgefragten Gesundheitsdaten in ein "Common Information Model" (312), wobei das "Common Information Model" durch mindestens einen Patienteneintrag definiert ist, wobei jeder Patienteneintrag mehrere Attribute aufweist, und wobei das erste Schaltmodul konfiguriert ist, die abgefragten Gesundheitsdaten von den zwei oder mehr Datenquellen mit dem mindestens einen Patienteneintrag unter Verwendung eines Gesamtübereinstimmungswertes abzugleichen, wobei der Gesamtübereinstimmungswert auf einer Summe von zwei oder mehr gewichteten Werten basiert, die auf die Attribute angewendet werden, wobei die Attribute der abgefragten Gesundheitsdaten vorbestimmten Gewichtungen zugewiesen werden, die basierend auf der Wahrscheinlichkeit der Änderung während eines Patientenlebens bestimmt werden, und bei Übereinstimmung, die abgefragten Gesundheitsdaten mit einer eindeutigen Kennung für den Patienten in dem Gesundheitsdatensystem gekennzeichnet werden; und
ein oder mehrere Schnittstellenmodule, um basierend auf einem Satz von Zugriffsberechtigungen Zugriff auf das "Common Information Model" zu erlangen.

2. Gesundheitsdatensystem nach Anspruch 1, wobei das "Common Information Model" als eine verteile Datenbank gespeichert ist, und wobei das eine oder die mehreren Attribute wahlweise klinische Gesundheitsdaten, Labordaten, Fernüberwachungsdaten, biometrische Daten, tragbare Geräte, Daten aus sozialen Medien, selbstberichtete Daten, Daten mobiler Anwendungen und/oder Vorrichtungsinstrumentierung definieren.

3. Gesundheitsdatensystem nach Anspruch 1 oder Anspruch 2, wobei das erste Schaltmodul (311) dem "Common Information Model" ein neues Attribut hinzufügt, wenn die abgefragten Gesundheitsdaten nicht in das eine oder die mehreren Attribute abgebildet werden.

4. Gesundheitsdatensystem nach einem der Ansprüche 1-3, wobei das erste Schaltmodul (311) zumindest einen Datensatz der abgefragten Gesundheitsdaten vor dem Bereitstellen der abgefragten Gesundheitsdaten in das "Common Information Model" basierend auf Speicherberechtigungen filtert, wobei die Speicherberechtigungen wahlweise durch den Gesundheitsdatenserver und/oder die eine oder die mehreren Gesundheitsdatenquellen bereitgestellt werden, und die Netzverbindungen über das gesicherte Netz steuert.

5. Gesundheitsdatensystem nach einem der Ansprüche 1-4, wobei die vorgesehene Frequenz durch den Gesundheitsdatenserver (120) und/oder der einen oder den mehreren Gesundheitsdatenquellen (110) eingestellt wird.

6. Verfahren zum Liefern von patientenzentrierter und wertbasierter Versorgung, wobei das Verfahren Folgendes umfasst:
Abfragen von zwei oder mehr Gesundheitsdatenquellen (110) nach Gesundheitsdaten über ein oder mehrere Agentenmodule (205) eines Gesundheitsdatenservers (120), wobei jede der einen oder mehreren Gesundheitsdatenquellen einen Satz von Abfrageberechtigungen aufweist, und wobei sich die zwei oder mehr Gesundheitsdatenquellen (110) über ein gesichertes Netz (140) in Kommunikation mit dem Gesundheitsdatenserver befinden, und wobei das Abfragen mit einer vorgesehenen Frequenz basierend auf einem Satz von Kennzeichen und dem Satz von Abfrageberechtigungen durchgeführt wird; und wobei die Datenquellen Körperüberwachungsvorrichtungen umfassen;
Befüllen eines "Common Information Model" mit den abgefragten Gesundheitsdaten über ein erstes Schaltmodul (311), wobei das "Common Information Model" durch mindestens einen Patienteneintrag definiert ist, wobei jeder Patienteneintrag ein oder mehrere Attribute aufweist, und wobei das Verfahren das Abgleichen der von den zwei oder mehr Datenquellen abgefragten Gesundheitsdaten mit dem mindestens einen Patienteneintrag unter Verwendung eines Gesamtübereinstimmungswerts enthält, wobei der Gesamtübereinstimmungswert auf einer Summe von zwei oder mehr gewichteten Bewertungen, die auf die Attribute angewendet werden, basiert, wobei die Attribute der abgefragten Gesundheitsdaten vorbestimmten Gewichtungen zugewiesen werden, die basierend auf der Wahrscheinlichkeit einer Änderung während eines Patientenlebens bestimmt werden, und bei Übereinstimmung, die abgefragten Gesundheitsdaten mit einer eindeutigen Kennung für den Patienten in dem Gesundheitsdatensystem markiert werden; und
Bereitstellen von Zugriff auf das "Common Information Model" über ein oder mehrere Schnittstellenmodule basierend auf einem Satz von Zugriffsberechtigungen.

7. Verfahren nach Anspruch 6, wobei das Befüllen des "Common Information Model" das Befüllen einer verteilten Datenbank wahlweise mit dem einen oder den mehreren Attributen umfasst, die aus klinischen Gesundheitsdaten, Labordaten, Fernüberwachungsdaten, biometrischen Daten, tragbaren Vorrichtungen, Daten aus sozialen Medien, selbstberichtete Daten, Daten mobiler Anwendungen und/oder Vorrichtungsinstrumentierung ausgewählt werden.

8. Verfahren nach Anspruch 6 oder Anspruch 7, das ferner das Hinzufügen eines neuen Attributes zu dem "Common Information Model" über das erste Schaltmodul umfasst, wenn die abgefragten Gesundheitsdaten nicht in das eine oder die mehreren Attribute abgebildet werden.

9. Verfahren nach einem der Ansprüche 6-8, das ferner das Filtern der abgefragten Gesundheitsdaten, basierend auf einem Satz von Speicherberechtigungen, vor dem Befüllen umfasst.

10. Verfahren nach einem der Ansprüche 6-9, wobei der Satz von Speicherberechtigungen durch den Gesundheitsdatenserver und/oder die eine oder mehreren Gesundheitsdatenquellen bereitgestellt wird.

11. Verfahren nach einem der Ansprüche 6-10, wobei die Abfragefrequenz durch den Gesundheitsdatenserver und/oder die eine oder die mehreren Gesundheitsdatenquellen eingestellt wird.

## Revendications

1. Système de données de santé (100) destiné à la prestation de soins centrés sur le patient et basés sur la valeur, comprenant :
un serveur de données de santé (120) ;
deux ou plusieurs sources de données de santé (110) en communication avec le serveur de données de santé sur un réseau sécurisé (140), lesdites sources de données de santé comportant chacune un ensemble d'autorisations d'interrogation et lesdites sources de données comprenant des dispositifs de surveillance corporels ;
un ou plusieurs modules agents (205) du serveur de données de santé qui interrogent des données de santé auprès des sources de données à une fréquence désignée en fonction d'un ensemble d'identifiants et de l'ensemble de permissions d'interrogation ;
un premier module de commutation (311) destiné à fournir les données de santé interrogées dans un modèle d'informations communes (312), le modèle d'informations communes étant défini par au moins un dossier de patient, chaque dossier de patient comportant une pluralité d'attributs, et dans lequel le premier module de commutation est configuré pour établir une concordance des données de santé interrogées provenant des deux ou plusieurs sources de données avec l'au moins un dossier de patient en utilisant un score de concordance total, le score de concordance total étant basé sur une somme de deux ou plusieurs scores pondérés appliqués aux attributs, dans lequel les attributs des données de santé interrogées se voient attribuer des poids prédéterminés déterminés en fonction de la probabilité de changement durant la vie d'un patient et, lors de la concordance, les données de santé interrogées sont étiquetées avec un identifiant unique pour le patient dans le système de données de santé ; et
un ou plusieurs modules d'interface pour obtenir l'accès au modèle d'informations communes en fonction d'un ensemble d'autorisations d'accès.

2. Système de données de santé selon la revendication 1, dans lequel le modèle d'informations communes est mémorisé sous forme de base de données distribuée et dans lequel les un ou plusieurs attributs définissent facultativement des données de santé cliniques, des données de laboratoire, des données de surveillance à distance, des données biométriques, des données de technologie vestimentaire, des données de media sociaux, des données auto-rapportées, des données d'applications mobiles, et/ou une instrumentation de dispositif.

3. Système de données de santé selon la revendication 1 ou la revendication 2, dans lequel ledit premier module de commutation (311) augmente le modèle d'informations communes avec un nouvel attribut quand les données de santé interrogées ne concordent pas avec les un ou plusieurs attributs.

4. Système de données de santé selon l'une quelconque des revendications 1 à 3, dans lequel ledit premier module de communication (311) filtre les données de santé interrogées avant de les fournir dans le modèle d'informations communes en fonction d'autorisations de mémorisation, les autorisations de mémorisation étant fournies facultativement par le serveur de données de santé et/ou les une ou plusieurs sources de données de santé, et/ou commande des connexions de réseau sur le réseau sécurisé.

5. Système de données de santé selon l'une quelconque des revendications 1 à 4, dans lequel la fréquence désignée est réglée par le serveur de données de santé (120) et/ou les une ou plusieurs sources de données de santé (110).

6. Procédé de prestation de soins centrés sur le patient et basés sur la valeur, le procédé comprenant :
l'interrogation de deux ou plusieurs sources de données de santé (110) à la recherche de données de santé par l'intermédiaire d'un ou plusieurs modules agents (205) d'un serveur de données de santé (120), chacune une ou plusieurs sources de données de santé ayant un ensemble d'autorisations d'interrogations et dans lequel les deux ou plusieurs sources de données de santé (110) sont en communication avec le serveur de données de santé sur un réseau sécurisé (140), et dans lequel ladite interrogation est réalisée à une fréquence désignée en fonction d'un ensemble d'identifiants et de l'ensemble d'autorisations d'interrogation ; et dans lequel lesdites sources de données comprennent des dispositifs de surveillance corporels ;
la population d'un modèle d'informations communes avec les données de santé interrogées par l'intermédiaire d'un premier module de commutation (311), le modèle d'informations communes étant défini par au moins un dossier de patient, chaque dossier de patient comportant un ou plusieurs attributs, et le procédé comportant l'établissement d'une concordance des données de santé interrogées provenant des deux ou plusieurs sources de données avec l'au moins un dossier de patient en utilisant un score de concordance total, le score de concordance total étant basé sur une somme de deux ou plusieurs scores pondérés appliqués aux attributs, dans lequel les attributs des données de santé interrogées se voient attribuer des poids prédéterminés déterminés en fonction de la probabilité de changement durant la vie d'un patient et, lors de la concordance, les données de santé interrogées sont étiquetées avec un identifiant unique pour le patient dans le système de données de santé ; et
la fourniture de l'accès au modèle d'informations communes par l'intermédiaire d'un ou plusieurs modules d'interface en fonction d'un ensemble de permissions d'accès.

7. Procédé selon la revendication 6, dans lequel ladite population du modèle d'informations communes comprend la population d'une base de données distribuée facultativement avec les un ou plusieurs attributs sélectionnés parmi des données de santé cliniques, des données de laboratoire, des données de surveillance à distance, des données biométriques, des données de technologie vestimentaire, des données de media sociaux, des données auto-rapportées, des données d'applications mobiles, et/ou une instrumentation de dispositif.

8. Procédé selon la revendication 6 ou la revendication 7, comprenant en outre l'augmentation du modèle d'informations communes avec un nouvel attribut quand les données de santé interrogées ne concordent pas avec les un ou plusieurs attributs par l'intermédiaire du premier module de commutation.

9. Procédé selon l'une quelconque des revendications 6 à 8, comprenant en outre le filtrage des données de santé interrogées en fonction d'un ensemble d'autorisations de mémorisation avant ladite population.

10. Procédé selon l'une quelconque des revendications 6 à 9, dans lequel l'ensemble d'autorisations de mémorisation est fourni par le serveur de données de santé et/ou les une ou plusieurs sources de données de santé.

11. Procédé selon l'une quelconque des revendications 6 à 10, dans lequel ladite fréquence d'interrogation est réglée par le serveur de données de santé et/ou les une ou plusieurs sources de données de santé.
